(19) Europäisches Patentamt
European Patent Office
Office européen des brevets

(11) EP 2 233 121 A1

(12) **EUROPEAN PATENT APPLICATION**

(43) Date of publication:
**29.09.2010 Bulletin 2010/39**

(21) Application number: **10250531.0**

(22) Date of filing: **22.03.2010**

(51) Int Cl.:
***A61H 23/02*** *(2006.01)* ***A61H 23/04*** *(2006.01)*
***A61H 1/00*** *(2006.01)* ***A61B 5/0452*** *(2006.01)*
***A61M 21/02*** *(2006.01)*

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(30) Priority: **24.03.2009 JP 2009071494**

(71) Applicant: **Sanyo Electric Co., Ltd.**
**Osaka 570-8677 (JP)**

(72) Inventor: **Hashimoto, Masahiko**
**Moriguchi-shi, Osaka 570-8677 (JP)**

(74) Representative: **Calderbank, Thomas Roger et al**
**Mewburn Ellis LLP**
**33 Gutter Lane**
**London**
**EC2V 8AS (GB)**

(54) **Massage apparatus and massage program**

(57) A massage apparatus includes: a massage unit configured to massage a user; a biological information acquisition unit configured to acquire biological information of the user; a stress estimation unit configured to estimate a degree of stress of the user based on the biological information acquired by the biological information acquisition unit; and an operation determination unit configured to determine a massage operation to be performed for the user by the massage unit based on the degree of stress of the user estimated by the stress estimation unit.

FIG. 1A

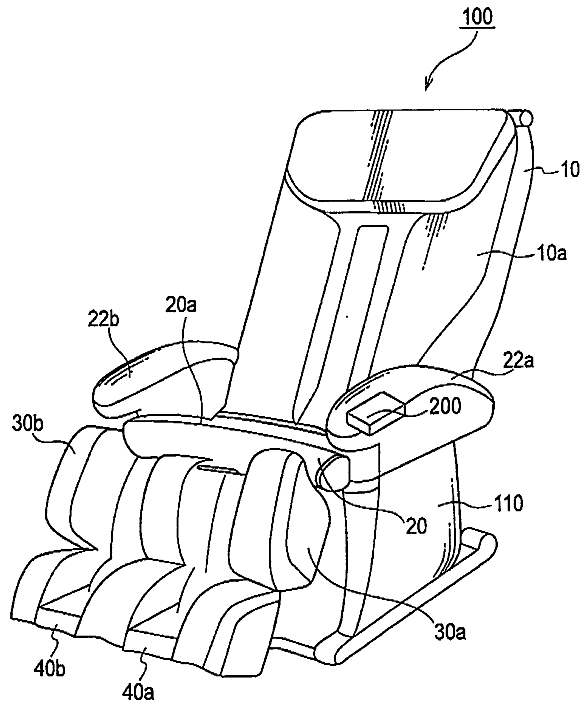




Printed by Jouve, 75001 PARIS (FR)

(Cont. next page)

FIG. 1B

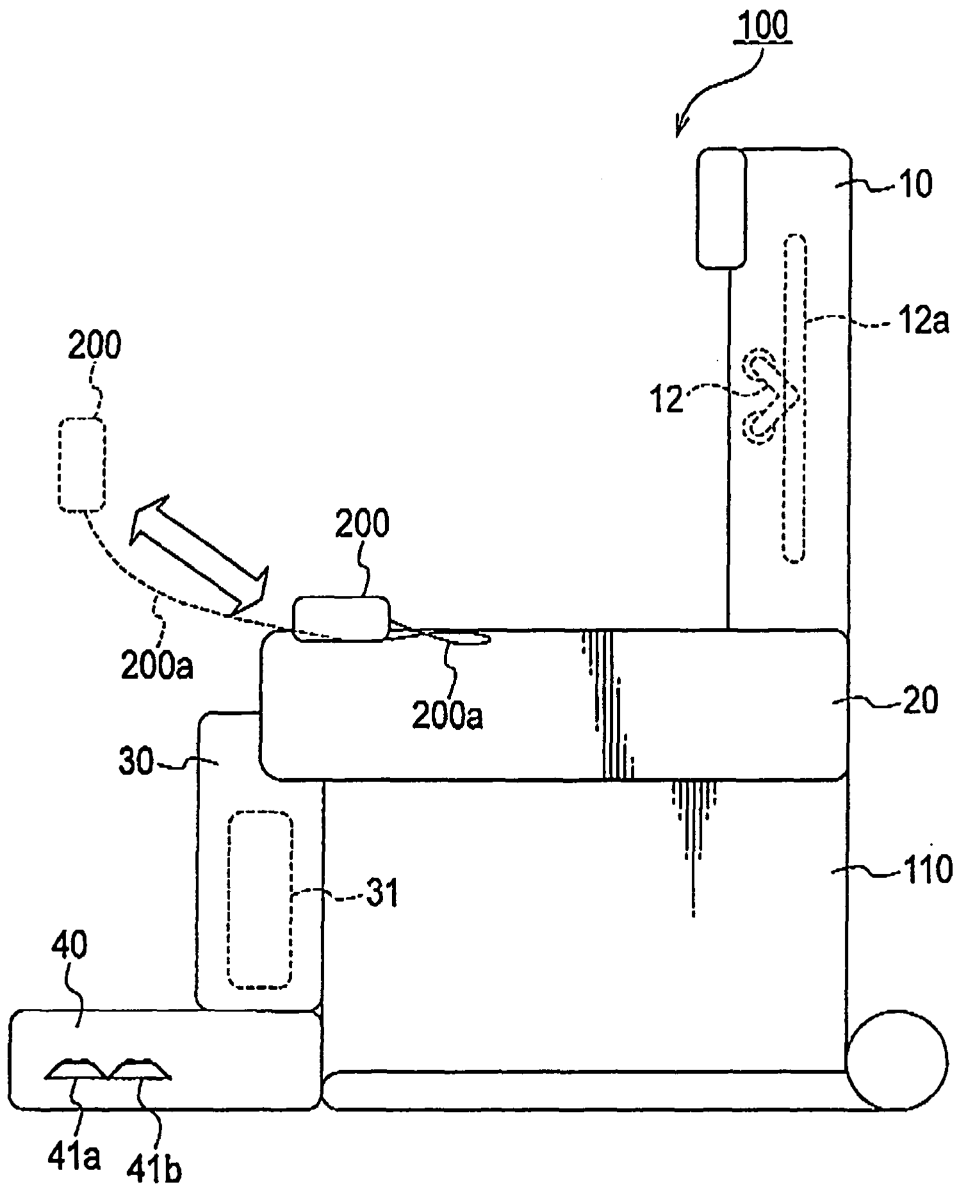

## Description

The present invention relates to a massage apparatus and a massage program which select a massage course to be performed for a user based on the user's degree of stress.

Heretofore, massage apparatuses have been used to massage users. For example, some known apparatuses are designed to massage each body part such as the shoulders, waist, back or the like, whereas some other known apparatuses are designed in the form of a bed to massage the entire back side of a user. Among them, a massage chair is widely known which includes: a seat portion on which a user is seated; a backrest portion supporting the back of the user seated on the seat portion and massaging the user; a footrest portion supporting the curves of the user; and a sole portion supporting the soles of the user. The massage chair massages the user by applying pressure to user's parts such as the neck, shoulders, back, waist, hips, thighs, curves, and soles. To perform such massages for the users, the massage chair is provided with massage courses in advance. Specifically, each massage course is predetermined as to which part of the body, how much (time and strength of the massage) and which type of massage operation is performed on. The types of massage operations are a kneading operation, an acupressure operation, a patting operation, a patting and kneading operation, and a rolling operation (a stroking operation with kneading balls).

Such a massage chair has a reclining function to adjust tilts of the backrest portion, the footrest portion, and the like and is configured so that the user can be massaged in a relaxed state. Relaxing the user leads to an increase in effect of the massage.

In order to massage the user in a relaxed state, types of massage operations, parts to be massaged, massage time and strength, and the like in addition to the tilts of the backrest and footrest portions of the massage chair need to be considered so as to be suitable for the user at that time.

Apparatus is proposed which includes a relaxing course of inducing a massage subject to a relaxed state based on judgment as to whether the massage subject is relaxed, and which is configured to display contents of a selected operation specified through an operation instruction switch and information concerning the massage operation including the positions of kneading balls and the like (see Japanese Patent Application Publication No. 2005-341989).

The aforementioned conventional art provides a relaxing course to perform massage inducing a massage subject to a relaxed state. However, in the aforementioned conventional art, the stress of the massage subject (hereinafter, also referred to as a user) may not be reduced.

A massage apparatus of first aspect includes: a massage unit configured to massage a user; a biological information acquisition unit configured to acquire biological information of the user; a stress estimation unit configured to estimate a degree of stress of the user based on the biological information acquired by the biological information acquisition unit; and an operation determination unit configured to determine a massage operation to be performed for the user by the massage unit based on the degree of stress of the user estimated by the stress estimation unit.

According to the first aspect, the massage apparatus includes the stress estimation unit configured to estimate the user's degree of stress based on the user's biological information and the operation determination unit configured to determine the massage operation to be performed for the user based on the user's degree of stress estimated by the stress estimation unit. Therefore, the massage apparatus is able to massage a user according to the user's degree of stress.

The biological information of the user acquired by the biological information acquisition unit may be information concerning heart rate. The apparatus may further include: a heart-rate information processing calculation unit configured to detect heartbeat period of the user from the information concerning heart rate and calculate a heart rate variability coefficient using the detected heartbeat period, the heart rate variability coefficient being a value related to a degree of fluctuation of the heartbeat period. The stress estimation unit may estimate the degree of stress of the user based on the heart rate variability coefficient calculated by the heart-rate information processing calculation unit. Thus, the massage apparatus is able to accurately estimate the user's degree of stress, and to massage the user according to the user's degree of stress.

The heart-rate information processing calculation unit may include: a change amount calculating unit configured to calculate a slope value of a linear function obtained by approximating data of the heartbeat period on a time axis for a predetermined period of time; and a valid region detecting unit detecting, as a valid region, a region which is a part or all of the predetermined period of time when the slope value calculated by the change amount calculating unit is judged to be equal to or less than a threshold. The heart-rate information processing calculation unit may calculate the heart rate variability coefficient using the heartbeat period within the valid region detected by the valid region detection portion. Thus, the massage apparatus is able to accurately estimate the user's degree of stress by using only valid data, and to massage the user according to the user's degree of stress.

The massage unit may start to perform a preliminary initial massage for the user before the operation determination unit determines the massage operation to be performed for the user.

The massage apparatus may further include: a massage controlling unit configured to control a massage for the user in a massage process from start of the massage to end of the massage; and an output unit configured to output information concerning stress of the user which is outputted from the massage controlling unit. The massage controlling unit may output the information concerning stress of the user to the output unit during the massage process or after the massage process is finished.

A massage program of second aspect directs a processor coupled to a massage apparatus that further comprises at least one biological information input and one or more motors. The massage program includes the steps of: inputting electrical data representing biological information of the user, into the processor; storing the inputted electrical data; comparing the inputted electrical data via at least one of a stored table of values, and generating an estimated stress level of the user; selecting a motor activation routine based on the estimated stress level; activating the one or more motors according to the selected activation routine.

A massage apparatus of another aspect includes: a massage unit configured to massage a user; a massage controlling unit controlling the massage for the user in a massage process from start of the massage to end of the massage; a biological information acquisition unit configured to acquire biological information of the user; a stress estimation unit configured to estimate a degree of stress of the user based on the biological information acquired by the biological information acquisition unit before the massage process is started; and a course selection unit selecting a massage course to be performed for the user based on the user's degree of stress estimated by the stress estimation unit before the massage process is started.

A massage chair of another aspect includes: a seat portion on which a user is seated; a backrest portion supporting the back of the user seated on the seat portion and configured to massage the user; and a massage controlling unit configured to control the massage for the user in a massage process from start of the massage to end of the massage, includes: a biological information acquisition unit configured to acquire biological information of the user seated on the seat portion; a stress estimation unit configured to estimate a degree of stress of the user based on the biological information acquired by the biological information acquisition unit before the massage process is started; a course selection unit configured to select a massage course to be performed for the user based on the user's degree of stress estimated by the stress estimation unit before the massage process is started; and an output unit configured to provide an output indicating that the massage course is selected, when the course selection selects the massage course to be performed for the user.

In the drawings:

Fig. 1 is a view showing an appearance of a massage chair according to an embodiment.
Fig. 2 is a functional block diagram showing a configuration of the massage chair according to the embodiment.
Fig. 3 is a functional block diagram showing the configuration of a heart-rate information processing block
Fig. 4 is an explanatory view for detection of valid regions according to the embodiment.
Fig. 5 is a calculation flowchart in the heart-rate information processing block according to the embodiment.
Fig. 6 is a functional block diagram showing a configuration of a stress estimator according to the embodiment.
Fig. 7 is a functional block diagram showing a configuration of a massage course selector according to the embodiment.
Fig. 8 is an explanatory view for massage course selection according to the embodiment.
Fig. 9 is a functional block diagram showing a configuration of a massage controlling unit according to the embodiment.
Fig. 10 is a functional block diagram showing a configuration of a massage course storage according to the embodiment.
Fig. 11 is a flowchart of a massage operation according to the embodiment.
Fig. 12 is a diagram showing experimental results.
Fig. 13 is a diagram showing other experimental results.

Hereinafter, an embodiment of the present invention is described with reference to the drawings.

The drawings are schematic, and it should be noted that dimensional proportions and the like are different from real ones. Accordingly, specific dimensions and the like should be determined referring to the following description. Moreover, it is obvious that it is certain that some portions have different dimensional relations or different proportion in the drawings.

This embodiment is described with a massage chair as an example of massage apparatuses. The hollowing requirements for the type of massage operation, parts to be massaged, massage time and strength, and the like suitable for the user are not limited to the massage chair and are also required for other massage apparatuses.

(Configuration of Massage Chair)

Hereinafter, the configuration of the massage chair according to the embodiment of the present invention is described with reference to the drawings. Figs. 1A and 1B are views showing an appearance of a massage chair 100 according to the embodiment of the present invention.

As shown in the external perspective view of the massage chair 100 of Fig. 1A, the massage chair 100 includes a body portion 110, a backrest portion 10, a seat portion 20, and a pair of footrest portions 30a and 30b (sometimes collectively referred to as a footrest portion 30), and a pair of sole portions 40a and 40b (sometimes collectively referred to as a sole portion 40). The massage chair 100 is provided with a remote controller 200 with which a user operates action of the massage chair 100.

The body portion 110 supports the backrest portion 10, seat portion 20, footrest portion 30, and sole portion 40. The body portion 110 includes a later-described massage controlling unit which controls massages performed for the user by the massage chair 100 and the like. The body portion 110 includes a function of controlling tilts of the backrest portion 10, seat portion 20, footrest portion 30, and sole portion 40 (a reclining function).

The backrest portion 10 includes a backrest surface 10a supporting the back of the user and massages the shoulders, back, waist, and the like of the user.

The seat portion 20 includes a seating surface 20a on which the user is seated. The seating surface 20a includes a pair of armrest portions 22a and 22b (sometimes collectively referred to as armrest portions 22). The armrest portion 22a is provided with the remote controller 200.

The footrest portions 30a and 30b support left and right calves of the user, respectively, and massage the user's calves. The footrest portions 30a and 30b have a same configuration.

The sole portions 40a and 40b support left and right soles of the user, respectively, and massage the user's soles. The sole portions 40a and 40b have a same configuration.

As shown in Fig. 1B, the backrest portion 10 includes kneading balls 12 used for massaging the user's shoulder, back, and waist. The backrest portion 10 is able to give massages to the user with the kneading ball 12. The backrest portion 10 includes a path 12a through which the kneading balls 12 move up and down. The kneading balls 12 perform massage operations (activation routine of motors) including: a kneading operation of kneading the user's shoulders and the like; a grasping operation of grasping the user's shoulders and the like; a grasping and kneading operation of kneading the user's shoulders like grasping; an acupressure operation of slowly applying pressure to the user's shoulders and the like; a tapping operation of tapping the user's back and waist and the like; a tapping and kneading operation of tapping and kneading the user's back and waist and the like; and a rolling operation of stroking the user's back and waist and the like.

The footrest portion 30 includes an airbag 31 massaging the user's calf. The airbag 31 is filled with air to blow up and releases the air to shrink. The airbag 31 can therefore intermittently apply pressure to the user's calf.

The sole portion 40 includes a plurality of acupressure portions 41a and 41b (sometimes collectively referred to as a acupressure portion 41) massaging the user's sole. The acupressure portions 41a and 41b include protrusions applying pressure to acupuresure points of the user's sole. The protrusions move up and down to stimulate the acupuresure points of the sole.

The remote controller 200 is provided for the armrest portion 22 of the seat portion 20 as described above and is connected to the massage chair 100 through a wire 200a. The remote controller 200 is configured to be detachable from the armrest portion 22 of the seat portion 20.

The remote controller 200 exchanges signals with the massage chair 100 via the wire 200a. However, The remote controller 200 may exchange signals with the massage chair 100 using wireless techniques such as IrDa and Bluetooth (registered trademark).

The remote controller 200 includes: an input unit receiving an operations by the user; and an output unit including a display device such as lamps, LED, and LCD, for example, and an audio output device such as a speaker, for example. The input unit includes switches and a touch panel, for example. The remote controller 200 further includes a biological information acquisition unit configured to acquire information concerning the user's heart rate (hereinafter, referred to as heart-rate information).

The remote controller 200 may include either a single unit or a plurality of units. For example, the remote controller 200 may include a main remote controller provided for the armrest portion 22 and a handy sub-remote controller detachable from the massage apparatus. In this embodiment, the remote controller 200 includes the main and sub remote controllers.

(Functional Block of Massage Chair)

Hereinafter, the functional blocks showing the configuration of the massage chair according to the embodiment of the present invention is described with reference to the drawings. Fig. 2 is a functional block diagram showing the configuration of the massage chair 100 according to the embodiment of the present invention.

As shown in Fig. 2, the massage chair 100 includes the aforementioned backrest portion 10, seat portion 20, footrest portion 30, sole portion 40, body portion 110, and remote controller 200.

The backrest portion 10 includes a shoulder position sensor 11, the kneading balls 12, a kneading motor 14a, a patting motor 14b, an acupressure motor 14c, an elevation motor 14d, and the airbag 15. The kneading motor, patting motor, acupressure motor, elevation motor 14a to 14d are sometimes collectively referred to as a motor 14.

For example, the shoulder position sensor 11 is connected to the kneading balls 12. In a state where the user is seated on the massage chair 100 or where the user's back is supported by the backrest surface 10a, the shoulder position sensor 11 detects the position of the user's shoulders while the kneading balls 12 move up and down along the path 12a. The shoulder position sensor 11 then outputs the detected position of the shoulders as shoulder position information.

The kneading motor 14a includes, for example, a motor and a mechanism moving the kneading balls 12 horizontally (not shown). For example, the right and left kneading balls 12 are kept moving horizontally to implement the kneading operation. The right and left kneading balls 12 are moved so as to approach each other horizontally to implement the grasping operation. The combination of these operations implements the kneading and grasping operation. The patting motor 14b includes, for example, a motor and a mechanism alternately moving the kneading balls 12 vertically (not shown) to implement the patting operation. The acupressure motor 14c includes, for example, a motor and a mechanism simultaneously vertically pressing the kneading balls 12 slowly (not shown) to implement the acupressure operation. The elevation motor 14d includes a mechanism of moving the kneading balls 12 up and down along the path 12a (not shown) to implement upward and downward movement of the kneading balls 12 or the rolling operation. The other massage operations not individually described can be implemented by using the motors 14 and aforementioned mechanisms.

The backrest portion 10 further includes the airbag 15 massaging the user's shoulders, back, and waist. The airbag 15 is filled with air to blow up and releases the air to shrink. The airbag 15 can therefore intermittently apply pressure to the user's shoulders, back, and waist.

After the power is turned on, the massage chair of the embodiment performs the following series of operations under control of the massage controlling unit. The massage chair detects the user's shoulder position; acquires the user's biological information; estimates user's stress; automatically selects a massage course to be performed for the user; performs a massage treatment for the user; and terminates the massage treatment for the user when the massage course is finished. This series of operations is also referred to as a massage sequence below. Specifically, the massage treatment for the user includes a preliminary massage to estimate user's stiffness and a main massage which actually heals the user. In the massage treatment, the preliminary and main massages are repeated alternately for a predetermined number of times.

In this embodiment, the massage process starts from the start of the massage treatment for the user to the end of the massage treatment for the user. The massage process may include the entire massage sequence or may range between detection of the user's shoulder position by the shoulder position sensor 11 and the end of the massage treatment for the user.

The seat portion 20 includes the airbag 21 massaging hips, thighs, and the like of the user. The airbag 21 is filled with air to blow up and releases the air to shrink. The airbag 21 can therefore intermittently apply pressure to the hips, thighs, and the like of the user.

The footrest portion 30 includes the airbag 31. The airbag 31 massages each curve of the user as described above.

The sole portion 40 includes the acupressure portion 41. The acupressure portion 41 is configured to massage each sole of the user as described above.

The backrest portion 10 constitutes a massage unit massaging the user, and the seat portion 20, footrest portion 30, and sole portion 40 constitute the massage unit massaging the user.

The main remote controller 200m includes an input unit 220m and an output unit (hereinafter, referred to as a display unit) 230m.

The sub-remote controller 200s includes a biological information acquisition unit 210, an input unit 220s, and an output unit (hereinafter, referred to as a display unit) 230s. In this embodiment, the sub-remote controller 200s is gripped by user's hands.

The biological information acquisition unit 210 is a sensor detecting the biological information of the user. In this embodiment, the biological information acquisition unit 210 is a heart-rate sensor and is referred to as a heart-rate sensor 210. The heart-rate sensor 210 detects the heart-rate information (for example, heart-rate waveform or the like) of the user.

The heart-rate sensor 210 is, for example, a cardiac potential sensor which includes two or more pairs of electrodes and measures the cardiac potential from the potential difference between user's palms gripping the remote controller 200s.

The heart-rate sensor 210 transmits the heart-rate information detected by the heart-rate sensor 210 through a analog digital converter (ADC) to the body portion 110.

The biological information acquisition unit may detect, pulse rate, skin temperature, amount of sweating, electrodermal activity (EDA), brain wave, pulse wave, blood flow, blood pressure, body temperature, and the like as the biological information other than the heart rate.

The input unit 220, which the input units 220m and 220s are collectively referred to, includes various types of keys, a touch panel, and the like allowing the user to start and stop massages and select a massage course. The input unit 220 transmits operation signals corresponding to the keys pressed by the user to the body portion 110.

The display unit 230, which the display units 230m and 280s are collectively referred to, displays information including an announcement of the completion of automatic selection of a massage course, a stress value estimated before the massage treatment, the massage course performed for the user (for example, the name of a massage course), and remaining time. The display unit 230 displays information concerning the user's stress which is transmitted from the body portion 110 and outputted by the massage controlling unit 80. As described later, the display of the information concerning the user's stress may include, for example, the aforementioned degree of the user's stress, a change in the degree of stress, later-described evaluation sentences, results of evaluation of the massage effect (or, relief of user's stress) by the massage controlling unit 80. For example, the evaluation results include evaluations of "good", "poor", and "unchanged" concerning the change in user's stress. The display by the display unit 230 may include lightening a course lamp.

The body portion 110 includes a control unit including a heart-rate information processing calculating unit 50, a massage course selector 70, the massage controlling unit 80, and a stress estimator 90, a massage course storage 60, and a reclining controlling unit 120. These are only functional blocks concerning the massage operation of this embodiment, and the functional blocks within the body portion 110 are not limited thereto. The control unit may include the massage course storage 60 and reclining controlling unit 120.

The control unit is a functional block executed by a processor mounted on the massage chair 100, such as a CPU.

The heart-rate information processing calculating unit 50 calculates the heartbeat period and heart rate from the user's heart-rate information (electrical data after analog-digital conversion) obtained from the heart-rate sensor 210. In this embodiment, the heart-rate information processing calculating unit 50 calculates at least the heartbeat period and an amount of change in heartbeat period. Moreover, the heart-rate information processing calculating unit 50 compares the amount of change in heartbeat period with a threshold to calculate valid periods when the heart-rate information is valid, thus calculating a heart rate variability coefficient (HRV coefficient) using the heartbeat period within only the valid periods. The heart-rate information processing calculating unit 50 outputs the calculated value of the HRV coefficient to the stress estimator 90 at the succeeding phase. The heart-rate information processing calculating unit 50 corresponds to a biological information acquisition step in the program.

The details of the heart-rate information processing calculating unit 50 are described later (see Fig. 3).

The stress estimator 90 is configured to compare a threshold with the calculated value of the HRV coefficient obtained from the heart-rate information processing calculating unit 50 to calculate a stress estimation value. The stress estimator 90 outputs the calculated stress estimation value to the massage course selector 70 at the succeeding phase. The stress estimator 90 corresponds to a stress estimation step in the program.

The details of the stress estimator 70 are described later (see Fig. 6).

The massage course selector 70 is a kind of an operation determinating unit determining the massage operation performed for the user and has a function of selecting a certain massage course from predetermined massage courses. To be specific, the massage course selector 70 compares a threshold with the stress estimation value obtained from the stress estimator 90 and selects a massage course. When an operation signal is inputted through the input unit 220 of the remote controller 200 by the user, the massage course is selected according to the inputted operation signal. The selected massage course is outputted to the massage course storage 60 and massage controlling unit 80. The massage course selector 70 corresponds to an operation determination step in the program.

The details of the massage course selector 70 are described later (see Fig. 7).

The massage course storage 60 stores contents of the plurality of massages corresponding to each massage course and a massage course program related to each massage course. Herein, the massage course program is a program controlling the time taken to massage each part of the user, and strength and speed of the massage performed for each part of the user. The massage courses can be arbitrarily determined. This embodiment includes three massage courses: a course for reducing user's stress; a course for helping with stiffness; and a course for relieving user's fatigue.

The massage course storage 60 outputs the massage course program and the like to the massage controlling unit 80 according to the massage course outputted from the massage course selector 70.

The details of the massage course storage 60 are described later (see Fig. 10).

The massage controlling unit 80 controls massages at least during the massage process from the start to the end of the massage treatment for the user. In this embodiment, the massage controlling unit 80 controls massages and others after the power is turned on. Specifically, according to the massage course outputted from the massage course selector 70, the massage course program outputted from the massage course storage 60, and the like, the massage controlling unit 80 instructs the functional blocks related to the massages, such as the backrest portion 10, seat portion 20, footrest portion 30, sole portion 40, and reclining controlling unit 120 to perform the massage operation of the massage course. For example, according to the massage program, the massage controlling unit 80 controls the kneading balls 12, motors 14, airbags 15, 21, and 31, acupressure portion 41, and the like and performs reclining control. The massage controlling unit 80 corresponds to a massage step in the program.

The details of the massage controlling unit 80 are described later (see Fig. 9).

Under control by the massage controlling unit 80, the reclining controlling unit 120 performs the reclining control which adjusts the tilts of the backrest portion 10, seat portion 20, footrest portion 30, and sole portion 40 with respect to the body portion 110. To be specific, the reclining controlling unit 120 outputs control signals to actuators (not shown) which are provided in the backrest portion 10, seat portion 20, footrest portion 30, and sole portion 40 for angle adjustment. Each actuator is adjusted to an angle corresponding to the control signal with respect to the body portion 110.

(Configuration of Heart-Rate Information Processing Calculating unit 50)

Hereinafter, the configuration of the aforementioned heart-rate information processing calculating unit 50 is described with reference to the drawings. Fig. 3 is a functional block diagram showing the configuration of the heaft-rate information processing calculating unit 50 according to the embodiment of the present invention.

As shown in Fig. 3, the heart-rate information processing calculating unit 50 includes a heart-rate information acquiring unit 51, a heartbeat period calculating unit 52, a change amount calculating unit 53, a threshold storage 54, a valid region detecting unit 55, a HRV coefficient calculating unit 56, and an output unit 57.

The heart-rate information acquiring unit 51 receives the heart-rate information transmitted from the heart-rate sensor 210 of the sub-remote controller 200s. The heart-rate information transmitted to the body portion 110 may be stored in a memory within the body portion 110. In such a case, the heart-rate information acquiring unit 51 reads the heart-rate information stored in the memory. In this embodiment, the heart-rate information processing calculating unit 50 directly uses the heart-rate information transmitted from the heart-rate sensor 210 because storing the heart-rate information requires a large amount of memory.

The heartbeat period calculating unit 52 detects peaks of R wave from the cardiac potential waveform included in the heart-rate information and calculates the heart rate and heartbeat period from R-R intervals (RRI). The heart rate and heartbeat period may be calculated from peak intervals of T wave of the cardiac potential waveform or calculated from peak intervals of a pulse wave using the pulse waveform as the heart-rate waveform instead of the cardiac potential waveform.

The change amount calculating unit 53 calculates the amount of change in heartbeat period calculated by the heartbeat period calculating unit 52. The heartbeat period momentarily changes, and therefore the amount of change thereof is calculated.

The threshold storage 54 stores the threshold for the amount of change. The threshold is a predetermined value determined from various experimental results before shipping, an average of amounts of change which are calculated by the change amount calculating unit 53 when the user used the massage chair 100 before, or the like.

The valid region detecting unit 55 compares the amount of change in heartbeat period calculated by the change amount calculating unit 53 with the threshold for the amount of change stored in the threshold storage 54. The valid region detecting unit 55 then determines whether the heart-rate information obtained by the heart-rate sensor 210 is valid. If the heart-rate information is valid, the valid region detecting unit 55 detects a region where the heart-rate information is valid and then outputs the region.

The HRV coefficient calculating unit 56 calculates the HRV coefficient using the heartbeat period within the region which is judged to be valid by the valid region detecting unit 55 from the heartbeat period calculated by the heartbeat period calculating unit 52. In the calculation of the HRV coefficient, operation concerning the degree of fluctuation of the heartbeat period or the dispersion degree of values of the heartbeat period is performed. As shown in Figs. 4B and 4D later described, for example, the fluctuation of the heartbeat period means that values of RRI (sampling values) are not constant and dispersed in terms of the time axis. In this embodiment, the variance, standard deviation, and average of the heartbeat period are calculated for calculation of the HRV coefficient as described later. The HRV coefficient is a coefficient of variation CV in RRI in an electrocardiogram and is also called CVRR.

The output unit 57 outputs the HRV coefficient calculated by the HRV coefficient calculating unit 56 to the stress estimator 90.

Even if being in a relaxed state, not few people feel stress. The stressed state is therefore not antithetical to the relaxed state, and the stressed state and relaxed state are not intimately related to each other. Accordingly, it should not be said that a massage apparatus taking the relaxed state into consideration includes a function taking the stress into consideration.

Next, the method of detecting the valid regions in the heart-rate information processing calculating unit 50 is described.

Fig. 4 shows explanatory views for detection of the valid regions.

Fig. 4A shows a heart-rate waveform acquired from the heart-rate sensor 210. The shown waveform is the waveform of the cardiac potential of the user. The horizontal axis indicates time (msec), and the vertical axis indicates cardiac potential (mV). R in Fig. 4A indicates peak values of R-wave of the cardiac potential and appears periodically.

In terms of intervals between adjacent peak values R (hereinafter, referred to as RR intervals or RRI), a RRI waveform chart (a heart rate variability waveform) shown in Fig. 4B is obtained. The horizontal axis indicates time at which peak values R are obtained (sec), and the vertical axis indicates RRI (msec). The RRI values are shown in the form of an analogue waveform for easy understanding of the characteristic thereof although the actual RRI values are obtained as sampling values (discrete values) for individual RRI.

The RRI waveform chart shows an RRI waveform including frequency components called a respiratory component and frequency components called a blood pressure component at lower frequencies than that of the respiratory component, the respiratory and blood pressure components being superimposed on each other. The respiratory component includes components at 0.15 to 0.45 Hz and is also called a high frequency (HF) component. The blood pressure component includes components at 0.05 to 0.15 Hz and is also called a low frequency (LF) component. The respiratory-related RRI variation is used as an index of parasympathetic nerve activity. It is said that the larger the peak power value the lower the degree of stress can be judged to be. The smaller the peak power value the higher the degree of stress can be judged to be. If the RRI waveform of Fig. 4B is subjected to an FFT operation or the like, a frequency region characteristic diagram can be obtained like Fig. 4C. Fig. 4C shows the aforementioned respiratory component (HF component) and blood pressure component (HL component).

Fig. 4D shows a RRI waveform chart with the blood pressure-related RRI variation being neglected. The pulses in the RRI waveform due to the blood pressure-related RRI variation are smaller than those due to the respiratory-related RRI variation and are therefore negligible. If the user is stimulated by external disturbance or the like, the RRI is narrowed to produce distortion in the RRI waveform. In Fig. 4D, the distortion is shown in part where the respiratory-related RRI variation component is lower than the average of RRI values. Specifically, using the RRI sampling values within a predetermined period of time, the average level thereof is linearly approximated by a least square method to obtain the tilt thereof. The distortion is included in part where the obtained tilt is larger then a predetermined value (stored in the threshold storage 54). In this embodiment, the predetermined period of time is 12 sec.

The aforementioned distortion of the RRI waveform due to the stimulation by external disturbance and the like increases the error in calculation of the HRV coefficient and is therefore not used in the calculation thereof. The calculation of the HRV coefficient requires use of heartbeat period data measured when the user is in a resting state. Accordingly, the degree of stress cannot be accurately estimated when momentary stress due to massage stimulation occurs. For example, when the momentary stress increases the heart rate, the heartbeat period (RRI) is shortened, and the HRV coefficient is increased. It is therefore wrongly judged that the degree of stress is reduced. Accordingly, the part of the RRI waveform including the distortion is a region where the data is invalid (invalid region) and is not used.

In other words, the HRV coefficient can be calculated by judging the presence of temporary stress due to massage stimulation or the like from the amount of change in heart rate. By not using the invalid regions, only the steady stress can be estimated.

The aforementioned predetermined value is determined by experiments and past performances so as to secure the accuracy of the HRV coefficient to some extent.

The heart-rate information processing calculating unit 50 detects valid regions by the aforementioned procedure. In this embodiment, RRI data for 12 seconds is collectively handled as a group. It is detected wether RRI data for each 12 seconds is valid. The period of time for each group is not limited to 12 seconds but should be not less than about 10 seconds. This is because the blood pressure-related RRI variation occurs at a cycle of about 10 seconds (the frequency is around 0.1 Hz in Fig. 4C) and the period of time per group needs to be equal to or more than about 10 seconds in order to reduce the influence by the blood pressure-related RRI variation.

Next, the flow of calculating the HRV coefficient in each functional block within the heart-rate information processing calculating unit 50 is described.

Fig. 5A shows a calculation flow S1000. The calculation flow S1000 is started by power activation and then proceeds to step S1001.

In the step S1001, the heart-rate information acquiring unit 51 receives the heart-rate information.

In step S1002, the heartbeat period calculating unit 52 calculates the heartbeat period.

In step S1003, the change amount calculating unit 53 calculates the amount of change in heart rate. Specifically, The RRI waveform for 12 seconds is linearly approximated using the least square method to calculate the tilt thereof.

In step S1004, the valid region detecting unit 55 compares the predetermined value stored in the threshold storage 54 and the slope calculated in the step S1003. If the absolute value of the tilt is equal to or less than the predetermined value, the flow proceeds to step S1005, and the absolute value of the tilt is more than the predetermined value, the flow proceeds to step S1006.

In the step S1005, the RRI data for 12 seconds is judged to be valid. The valid region detecting unit 55 turns on a valid flag of the RRI data for 12 seconds (valid flag = 1) and notifies the HRV coefficient calculating unit 56. The flow then proceeds to the step S1007.

In the step S1006, the RRI data for 12seconds is judged to be invalid. The valid region detecting unit 55 turns off the valid flag of the RRI data for 12 seconds (valid flag=0) and notifies the HRV coefficient calculating unit 56. The flow then proceeds to the step S1007.

The calculated slope is compared with the predetermined value in the step S1004 for the following reason. The RRI waveform naturally includes the blood pressure-related RRI variation. When the RRI waveform is linearly approximated, the resultant waveform includes some slope due to the blood pressure-related RRI variation (the RRI blood pressure-related RRI variation component pulsates in a sinusoidal fashion at about 0.1 Hz as shown in Fig. 4B). Thus, the comparison of the calculated slope is for preventing a part including the blood pressure-related RRI variation component from being detected as the invalid region. Accordingly, the predetermined value is set to such a value that the part including the blood pressure-related RRI variation component will not be detected as the invalid region.

In the step S1007, the HRV coefficient calculating unit 56 calculates the HRV coefficient using later-described N RRI values judged to be valid and outputs the calculated HRV coefficient to the output unit 57. In this embodiment, the HRV coefficient is calculated by the following equation.

(Equation 1)

$$(\text{Vriance}) = \frac{\sum_{I=1}^{N}\left((\text{Heartbeat Period (i)} - (\text{Average Heartbeat Period})\right)^2}{N}$$

$$(\text{Standard Deviation}) = \text{Positive Square Root of Variance}$$

$$(\text{HRV Coefficient}) = \frac{\text{Standard Deviation}}{\text{Average Heartbeat Period}} \times 100[\%]$$

The heartbeat period (i) is an i-th heartbeat period (i=1 to N), and the average heartbeat period is an average of N heartbeat periods. N is a predetermined number determined by various experimental results before shipping, value obtained when the user used the massage chair 100 before, and the like. In this embodiment, N is 100, for example,

With reference to Fig. 5B, the way of using N RRI values from a group with the valid flag of 1 in the step S1007 is described.

As described above, in the embodiment, the RRI data for 12 seconds are handled as one group, and sequential groups A, B, C... for 12 seconds are considered. Herein, the adjacent ones of the groups A, B, C... are overlapped each other by four seconds.

It is considered whether the common portion of 4 seconds where the groups A, B, and C are overlapped each other (portion (α) in Fig. 5B) is valid. The N PRI values are extracted from a set of 4-seconds common portions judged to be valid.

If all of the groups A to C are judged to be valid in the steps S1004 and S1005, the overlapped four-seconds common portion (α) is judged to be valid. This is because the common portion (α) is included in only the groups judged to be valid.

However, if the group D is judged to be invalid in the steps S1004 and S1006, the overlapped four-seconds common portion (β) is judged to be invalid. This is because the common portion (β) is not included in only the groups judged to be valid or is included in the group D judged to be invalid. Similarly, the four-second common portions (γ) and (δ) are judged to be invalid.

In the case where the group E is judged to be valid in the steps S1004 and S1005 and the groups F and G not shown are also judged to be valid in the steps S1004 and S1005, the overlapped four-second common portion (ε) is judged to be valid. This is because the common portion (ε) is included in only the groups judged to be valid.

As described above, in the step S1007, the overlap portion of three groups which have a valid flag of 1 and are overlapped each other by four seconds is used. In the step S1007, the four-seconds common portions judged to be valid constitute a set, from which N RRI values are chronologically extracted for calculation of the heartbeat variability using the aforementioned Equation 1.

(Configuration of Stress Estimator 90)

In the following, the configuration of the aforementioned stress estimator 90 is described with reference to the drawings. Fig. 6 is a functional block diagram showing the configuration of the stress estimator 90 according to the embodiment of the present invention.

As shown in Fig. 6, the stress estimator 90 includes a HRV coefficient calculated value acquiring unit 91, a threshold storage 92, a stress estimation value calculating unit 93, and an output unit 94.

The HRV coefficient calculated value acquiring unit 91 is configured to acquire the value of the HRV coefficient outputted from the output unit 57.

The threshold storage 92 stores the threshold of the HRV coefficient (stored table of value) such as a predetermined value determined by results of various experiments before shipping, the average of values of the HRV coefficient calculated when the user used the massage chair 100 before, or the average of values of the HRV coefficient in a resting state obtained by past subject experiments.

The stress estimation value calculating unit 93 compares the HRV coefficient obtained by the HRV coefficient calculated value acquiring unit 91 with the threshold of the HRV coefficient stored in the threshold storage 92 and calculates the stress estimation value. In this embodiment, the stress estimation value is any one of 1 to 3. The stress estimation values of 1 to 3 correspond to evaluation sentences of "Your stress is very high", "Your stress is high", and "Your stress is low", respectively (see the examples of the stress estimation values and estimation results in Fig. 8).

The larger the HRV coefficient the more the stress can be judged to be reduced. The smaller the HRV coefficient the higher the degree of stress can be judged. Accordingly, by comparing the calculated HRV coefficient and the threshold, the degree of stress in the user's steady state is classified using the stress estimation value. This classification is described together with the description of the massage course selector 70 and Fig. 8.

The output unit 94 outputs the stress estimation value calculated by the stress estimation value calculation unit 93 to the massage course selector 70.

(Configuration of Massage Course Selector 70)

The configuration of the aforementioned massage course selector 70 is described below with reference to the drawings. Fig. 7 is a functional block diagram showing the configuration of the massage course selector 70 according to the embodiment of the present invention.

As shown in Fig. 7, the massage course selector 70 includes a stress estimation value acquiring unit 71, an operation signal acquiring unit 72, a threshold storage 73, a course selecting unit 74, and an output unit 75.

The stress estimation acquiring unit 71 is configured to acquire the stress estimation value outputted from the output unit 94.

The operation single acquiring unit 72 accepts operation signals concerning massage course selection which are inputted from the input unit 220 of the remote controller 200.

The threshold storage 73 stores the threshold of the stress estimation value such as a predetermined value determined by results of various experiments before shipping, the stress estimation value calculated when the user used the massage chair 100 before, or the like.

The course selecting unit 74 selects one of the massage courses according to the stress estimation value. When the degree of user's stress is high, the massage course effective on reducing stress is selected. The massage courses are previously determined by subject experiments. When the user specifies one of the courses, or when an operation signal concerning the course selection is inputted, a massage course is selected according to the specif cation by the operation signal.

The output unit 75 outputs the massage course selected by the course selecting unit 74 to the massage course storage 60.

With reference to Fig. 8, the relationship between the course selection based on the stress estimation result and the CVRR values in the course selecting unit 74.

When the calculated CVRR value is smaller, the degree of stress is judged to be high, and the course more effective on reducing the stress is selected. In this embodiment, when the calculated CVRR value is less than 3.5%, the stress is judged to be very high, and a course A, which is most effective on reducing the stress, is selected. When the calculated CVRR value is not less than 3.5% and less than 4.5%, the stress is judged to be high, and a course B which is second most effective on reducing the stress, is selected. When the calculated CVRR value is more than 4.5%, the stress is judged to be low, and a course C which is least effective on reducing the stress, is selected.

Fig. 8 also shows later-described experimental results.

An example of the operation of each course is shown below. The figures in blanks indicate proportions of time taken to conduct the individual operations in each course. The operations are not necessarily executed in the order of operations a, b, c, and d, and the others.

Course A: most effective in reducing stress.

operation a (40%) + operation b (30%) + operation c (10%) + operation d (10%) + others (10%)

Course B: prioritizes the massage effect including stiffness alleviation to the effect on reducing stress

operation a (30%) + operation b (20%) + operation c (20%) + operation d (20%) + others (10%)

Course C: less effective on reducing stress but effective on refreshing such as relieving fatigue

operation a (10%) + operation b (20%) + operation c (30%) + operation d (30%) + others (10%)

The operations a to d in the descending order of stress reducing effects are operation a > operation b > operation c > operation d. The larger the movement of the kneading balls 12 the lower the effect on reducing stress. This is because the larger movement of the kneading balls gives larger stimulus to the user and the sympathetic nerve becomes predominant to the parasympathetic nerve to increase the stress. For example, the operation a is the kneading operation; the operation b, the acupressure operation; the operation c, the patting operation; the operation d, the patting and kneading operation; and the other operations, the rolling operation. The operations a to d and the other operations are set at the massage chair 100 before shipping.

(Configuration of Massage controlling unit 80)

The configuration of the massage controlling unit 80 is described below with reference to the drawings. Fig. 9 is a functional block diagram showing the configuration of the massage controlling unit 80 according of the embodiment of the present invention.

As shown in Fig. 9, the massage controlling unit 80 includes a course specifying signal acquiring unit 81, a timer 82, a shoulder position information acquiring unit 83, a course operation controlling unit 84, an instruction unit 85, and an other operation controlling unit 86.

The course specifying signal acquiring unit 81 is configured to acquire the massage course outputted from the output unit 75 and the massage course program outputted from the program output unit 75 of the massage course storage 60.

The timer 82 outputs time information to the course operation controlling unit 84 or other functional sections since the time information is necessary to control the massage operations and the like.

The shoulder position information acquiring unit 83 acquires the shoulder position information outputted from the shoulder position sensor 11 of the backrest portion 10.

The course operation controlling unit 84 controls the massage operation performed for the user in the aforementioned massage sequence according to the acquired massage program. For example, the course operation controlling unit 84 outputs control information to the functional units to control the kneading balls 12, motors 14, airbags 15, 21, and 31 and performs reclining control.

The instruction unit 85 sends the control information from the course operation controlling unit 84 to the corresponding functional unit out of the backrest portion 10, seat portion 20, footrest portion, sole portion 40, and reclining controlling unit 120 for instruction.

The other operation controlling unit 86 controls operations of the massage chair 100 other than the massage operation in the massage sequence. For example, the other operation controlling unit 86 performs control between the massage chair 100 and the input and display units 220 and 230 of the remote controller 200, control concerning power supply, and the like.

(Configuration of Massage course storage 60)

The configuration of the massage course storage 60 is described below with reference to the drawings. Fig. 10 is a functional block diagram showing the configuration of the massage course storage 60 according to the embodiment of the present invention.

As shown in Fig. 10, the massage course storage 60 includes: a base address calculating unit 61, a storage 62, and a program output unit 63.

The base address calculating unit 61 acquires the massage course outputted from the output unit 75 and outputs a memory address at which the massage course is stored (a base address).

The storage 62 stores a program of each massage course. The storage 62 adds a relative address to the base address to read the massage course program corresponding to the massage course outputted from the output unit 75. The program includes operations included in the massage course, the motors, airbags, and the like used in the massage course, treatment time, strength, and the like.

The program output unit 63 outputs the read massage course program to the massage controlling unit 80.

(Entire Operation Flow)

Next, the series of massage operations performed for the user is described.

When the power is turned on, the operation proceeds to step S100.

In the step S100, the massage controlling unit 80 detects the shoulder position using the shoulder position sensor 11. According to the detected shoulder position, the positions of the back, waist, and the like are confirmed, and the user's massage treatment range is determined. Next, the flow proceeds to step S1000 and step S400.

The step S1000-1 is the above-described calculation of the HRV coefficient in the heart-rate information processing calculating unit 50 (steps S1001 to S1007). The HRV coefficient before the massage treatment is executed. Next, the flow proceeds to step S200-1.

In the step S200-1, the stress estimation value is calculated in the stress estimator 90. At this time, the display unit 230 displays the user's estimated stress state before the massage treatment according to the stress estimation value is executed. Next, the flow proceeds to step S300.

In the step S300, the massage course selector 70 selects a massage course according to the stress estimation value. At this time, the display unit 230 outputs completion of selection of the massage course. For example, the display unit 230 announces that the course selection is completed or displays the selected course. Alternatively, the display unit 230 may turned on a course lamp. The stress estimation value before the massage treatment may be displayed together in this step.

In the step S400, an initial massage is performed for the user as a pre-massage in parallel to the operations in the above-described steps S1000-1, S200-1, and S300 or until the massage course to be performed for the user is selected in the massage course selector 70 after the user's biological information starts to be acquired in the heart-rate sensor 210 before the massage controlling unit 80 starts to control the massage process. The initial massage is conducted in order to preliminarily place burden on the user's body before the massage treatment so as to prevent massage load from being rapidly applied to the user's body or in order to keep the user from being bored because it takes long time to acquire the heart-rate information, estimate stress, and select a massage course. The preliminary massage can be considered as an initial massage different from the main massage as long as the preliminary massage is conducted for the aforementioned purposes. The initial massage may be conducted before the massage course to be performed for the user is selected or after the shoulder position detection is started.

In this embodiment, the validity of the heart-rate information is judged as described above. The situation where the initial massage and the calculation of the HRV coefficient are performed in parallel to each other is one of the situations where the massage chair of this embodiment can exert the effects thereof. This is because the external stimulus by the initial massage will increase the frequency of occurrence of invalid heart-rate information compared to the normal cases.

In step S500, the massage treatment for the user starts according to the selected massage course. The performed massage includes the preliminary and main massages. The preliminary massage is performed in order to check stiffness of user's acupuresure points and entire back. The main massage actually heals the user according to the check results of the preliminary massage. The main and preliminary massages are handled in a pair and are each performed once. Thereafter, the flow proceeds to the step S1000-2.

The step S1000-2 is the above-described calculation of the HRV coefficient in the heart-rate information processing calculating unit 50. In the step S1000-2, the HRV coefficient during the massage treatment is calculated. Next, the flow proceeds to the step S200-2.

In the step S200-2, the stress estimation value is calculated in the stress estimator 90. At this time, the display unit 230 displays the estimated stress state of the user who is being massaged or the aforementioned evaluation sentence according to the stress estimation value. The display unit 230 may display the result from evaluation of the massage effect by the massage controlling unit 80, which is sent from the body portion 110. Next, the flow proceeds to step S600.

In the step S600, it is judged whether the massage treatment including, as a set, one preliminary massage and one main massage is finished. After the massage treatment is finished, it is judged whether a predetermined number of sets of the preliminary and main massages are completed. If the predetermined number of sets are executed, the flow proceeds to the step S1000-3. If the predetermined number of sets are not completed, the flow returns to the step S500. In this embodiment, the estimation of the stress state and display of the stress estimation value and the like in the step S200-2 are executed at any time any number of times. Accordingly, such estimation and display are not necessarily executed at each loop of the steps S500 to S600.

In this embodiment, the validity of the heart-rate information is judged as described above. The situation where the initial massage and the calculation of the HRV coefficient are performed in parallel to each other is one of the situations where the massage chair of this embodiment can exert the effects thereof. This is because the external stimulus by the initial massage will increase the frequency of occurrence of invalid heart-rate information compared to the normal cases.

The step S1000-3 is the above described calculation of the heart-rate information processing calculating unit 50 described above. The HRV coefficient after the massage treatment is calculated. Next, the flow proceeds to the step S200-3.

In the step S200-3, the stress estimation value is calculated. At this time, the display unit 230 displays the estimated stress state of the user or the aforementioned evaluation sentence after the massage treatment according to the stress estimation value. Moreover, the display unit 230 displays the stress state before the massage treatment (which can include the stress estimation value) and the stress state after the massage treatment (which can include the stress estimation value). This allows the user to know the degree of reduction of stress by the massage and the effect of relieving stress. The display 230 may display the results from evaluation of the massage effect by the massage controlling unit 80, which is sent from the body portion 110.

(Advantageous Effect)

According to the massage chair 100 of the embodiment of the present invention, in the heart-rate information processing operation block 50, the valid periods when the heart-rate information is valid are detected, and the HRV coefficient is calculated using the heartbeat period within only the valid periods. In the stress estimator 90, the stress estimation value is calculated. In the massage course selector 70, a massage course is selected for performing massage for the user. It is therefore possible to provide a massage apparatus and a massage program which estimates the user's degree of stress for selecting a massage course performed for the user and perform massage reducing stress for the user.

Moreover, the display unit 230 displays: announcement of end of automatic selection of a massage course; the stress value estimated before the massage treatment; the massage course to be performed for the user (including display of the name of the massage course and lighting of the course lamp); information including remaining time; and information on user's stress. The information on user's stress includes, for example: the user's degree of stress (for example, the aforementioned stress estimation value, the estimation sentences, and the like); a change in user's degree of stress, notification of the change; and evaluation of good, poor, and unchanged concerning the change in user's degree of stress by the massage controlling unit 80. Accordingly, it is possible to provide a massage apparatus and a massage program which estimate the user's degree of stress, select a massage course performed for the user, and massage the user, and output information that the massage course is selected.

Next, the description is given of experiments for confirming the effect of the massage chair 100 of the embodiment on reducing stress.

The experiments are outlined as follows.

Number of subjects: 18 (6 for each of the massage courses A to C)

Experiments: the subjects were treated with massages by the massage chair 100 of this embodiment, and the cardiac potentials were measured before and after the massage and during the massage course as the heart-rate information (sampling frequency=1 kHz).

The massage courses are:

(Massage Course A) providing an effect on reducing stress
(Massage Course B) providing massage effects including reduction of stiffness
(Massage Course C) providing refresh effects including fatigue relief.

The heart rate and HRV coefficient were calculated using the above measured cardiac potential sampling data.

Fig. 12 shows the experimental results (changes in heart rate). In terms of the change in heart rate, the difference between the heart rates at the start and end of each massage course did not significantly vary depending on the massage courses.

This revealed that the change in the HRV coefficient shown below were little influenced by the change in heart rate. Accordingly, it can be said that the massage chair 100 measures the steady change in stress.

Fig. 13 shows experimental results (change in HRV coefficient). As for the massage course A configured to have an effect on reducing stress, the HRV coefficient must be higher at the end of the massage course A than at the start thereof, and the experimental results showed the same. As for the massage course B configured to have a massage effect such as reduction of stiffness, the HRV coefficient at the end of the massage course B must be close to the start thereof, and the experimental results showed the same. As for the massage course C configured to have a refresh effect such as fatigue relief, the HRV coefficient must be lower at the end of the massage course C than at the start thereof, and the experimental results showed the same. The users were exposed to external disturbance during the massage treatment, and the user's degree of tension momentarily changed. The results during the massage course were different from those at the end of the massage course as shown in the same drawing.

Consequently, it can be said that the effects of stress reduction, stiffness reduction, and refresh can be provided for the user by calculating the CVRR value according to the degree of stress estimated before starting the massage course and automatically selecting one of the massage courses A to C.

Fig. 8 shows the numerical values by the experimental results in the line of HRV coefficient increase. The HRV coefficient increase is obtained by calculated HRV coefficient at the end of the course - calculated HRV coefficient at the start of the course.

As described above, the massage chair 100 of this embodiment was confirmed to have an effect on reducing stress.

From the above experimental results, the inventors decided to apply the CVRR to the massage apparatus and the massage program which estimates user's degree of stress and selects a massage course performed for the user.

(Other Embodiments)

As described above, the above embodiment is described using the massage chair as an example of the massage apparatus. It should be noted that the present invention disclosed in the above embodiment is not limited to the massage chair and is applicable to massage apparatuses other than the massage chair. Specifically, taking into consideration the fact that the description about the shoulder detection, reclining control of the backrest portions, footrest portions, and sole portions, and the like is specific to the massage chair, it can be easily thought up to apply the present invention to other massage apparatuses.

The above embodiment is described with the three massage courses as an example: the course for reducing stress, the course for reducing stiffness, and the course for relieving fatigue. However, the massage courses are not limited to these three.

Moreover, in the above embodiment, the HRV coefficient is calculated using the cardiac potential. However, vital data including pulse waves may be used as substitute data.

Furthermore, in the above embodiment, the initial massage and preliminary massage are performed in addition to the main massage. When, how often, and how these massages are performed are not limited to the example of the aforementioned embodiment, and these massages can be arbitrarily executed. The initial and preliminary massages do not need to be performed.

The hardware of the control unit in the embodiment can be implemented by a CPU, a memory, and other LSI of an arbitrary computer. It goes without saying that the functional blocks thereof can be implemented in various forms, such as only hardware, only software, or combinations thereof. The control unit is shown by the functional blocks in Fig. 2 and therefore tends to be known as being hardware. However, by considering replacement of the functional blocks with functional steps, the control unit can be easily known as being software. Software operation flows thereof can be easily known by replacing the massage operation of Figs. 5 and 11 with software controls.

The embodiments of the present invention can be properly variously changed within a technical scope described in claims. The aforementioned embodiments are just embodiments of the present invention, and the meaning of the terms of the present invention and each constituent element are not limited to the description of the above embodiments.

## Claims

**1.** A massage apparatus comprising:

a massage unit configured to massage a user;
a biological information acquisition unit configured to acquire biological information of the user;
a stress estimation unit configured to estimate a degree of stress of the user based on the biological information acquired by the biological information acquisition unit; and
an operation determination unit configured to determine a massage operation to be performed for the user by the massage unit based on the degree of stress of the user estimated by the stress estimation unit.

**2.** The massage apparatus according to claim 1, wherein
the biological information of the user acquired by the biological information acquisition unit is information concerning heart rate,
the apparatus further comprising:

a heart-rate information processing calculation unit configured to detect heartbeat period of the user from the information concerning heart rate and calculate a heart rate variability coefficient using the detected heartbeat period, the heart rate variability coefficient being a value related to a degree of fluctuation of the heartbeat period, wherein
the stress estimation unit estimates the degree of stress of the user based on the heart rate variability coefficient calculated by the heart-rate information processing calculation unit.

**3.** The massage apparatus according to claim 2, wherein
the heart-rate information processing calculation unit includes:

a change amount calculating unit configured to calculate a slope value of a linear function obtained by approximating data of the heartbeat period on a time axis for a predetermined period of time; and
a valid region detecting unit detecting, as a valid region, a region which is a part or all of the predetermined period of time when the slope value calculated by the change amount calculating unit is judged to be equal to or less than a threshold, and

the heart-rate information processing calculation unit calculates the heart rate variability coefficient using the heartbeat period within the valid region detected by the valid region detection portion.

**4.** The massage apparatus according to claim 1, wherein
the massage unit starts to perform a preliminary initial massage for the user before the operation determination unit determines the massage operation to be performed for the user.

**5.** A massage program that directs a processor coupled to a massage apparatus that further comprises at least one biological information input and one or more motors, comprising the steps of:

inputting electrical data representing biological information of the user, into the processor;
storing the inputted electrical data;
comparing the inputted electrical data via at least one of a stored table of values, and generating an estimated stress level of the user;
selecting a motor activation routine based on the estimated stress level;
activating the one or more motors according to the selected activation routine.

FIG. 1A

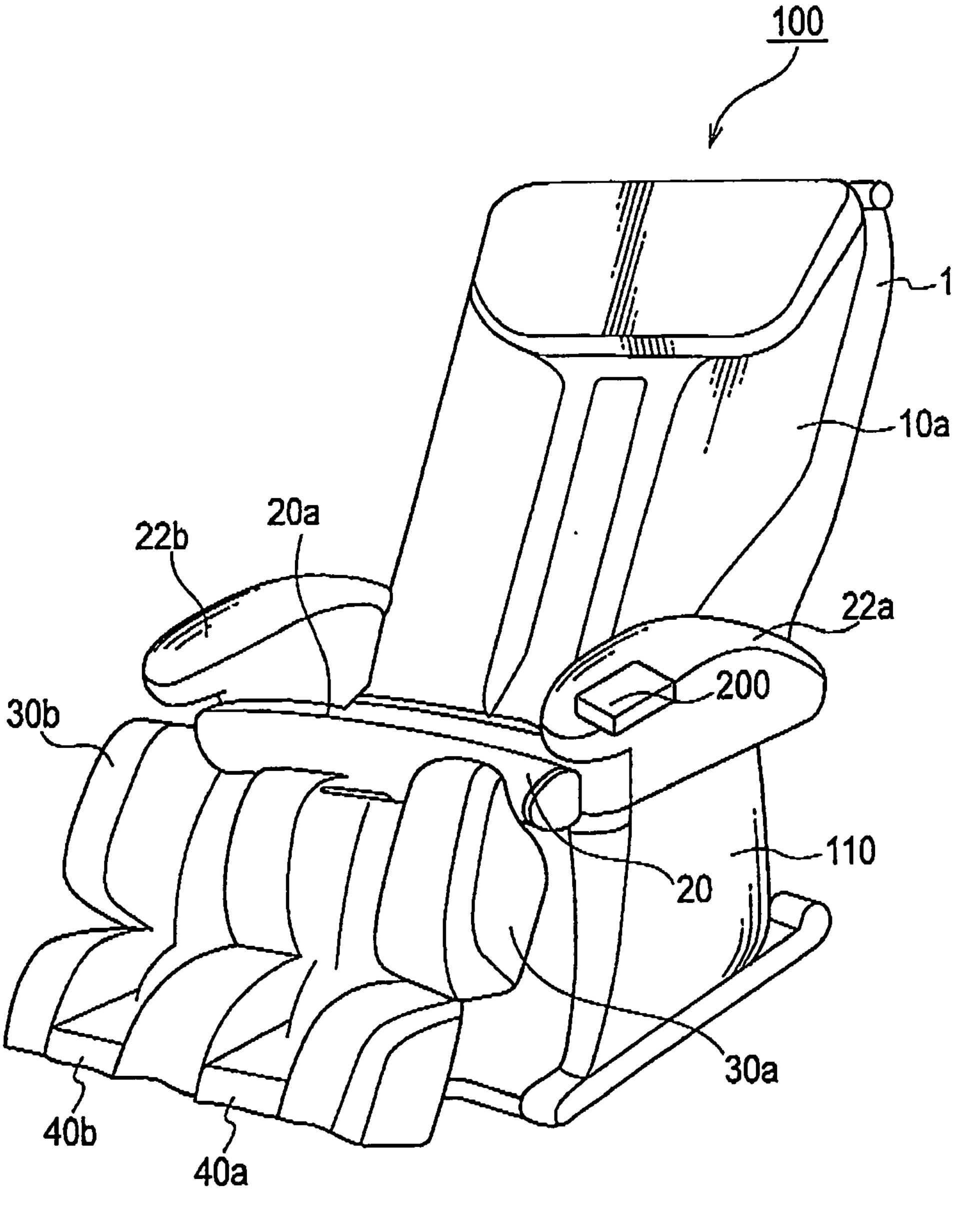

FIG. 1B

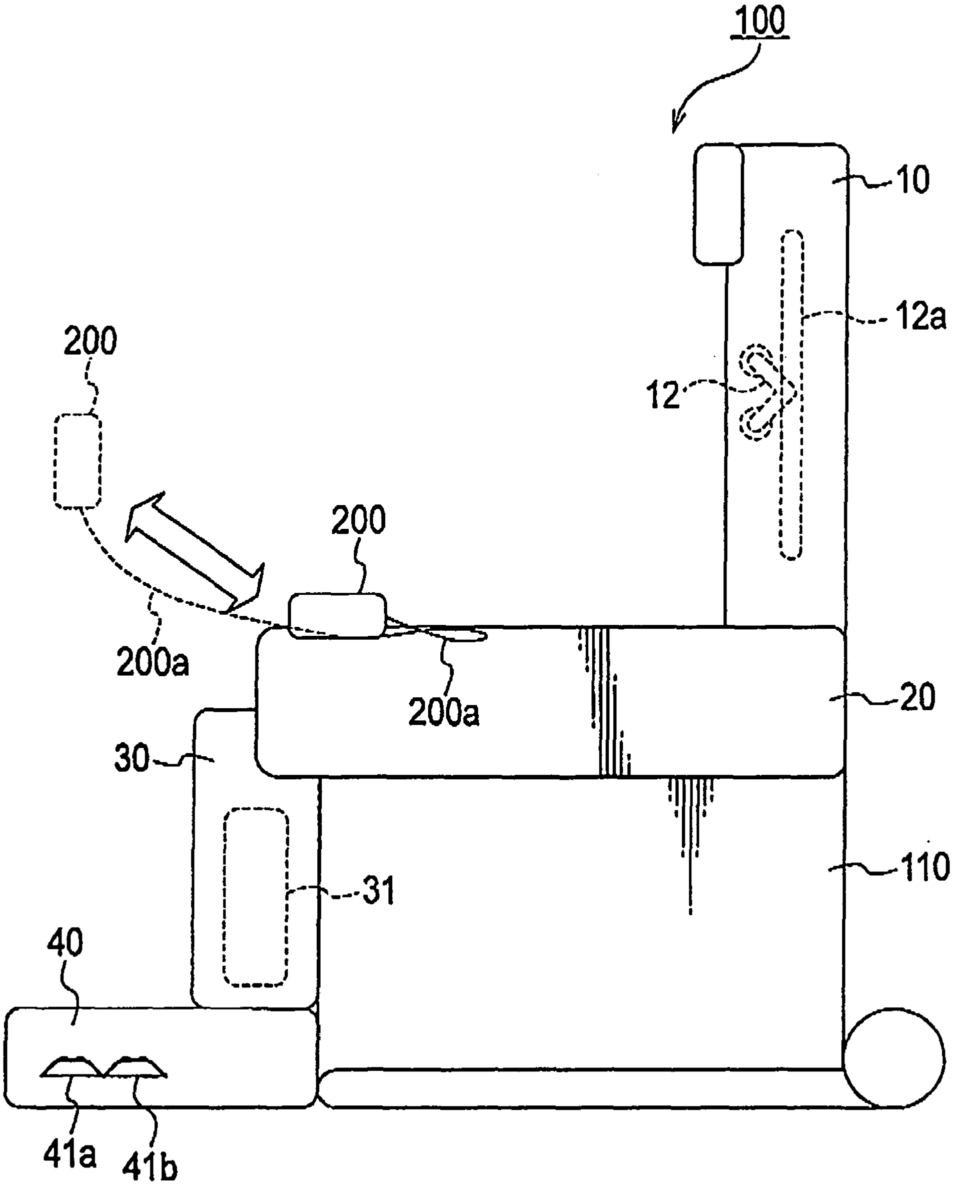

FIG. 2

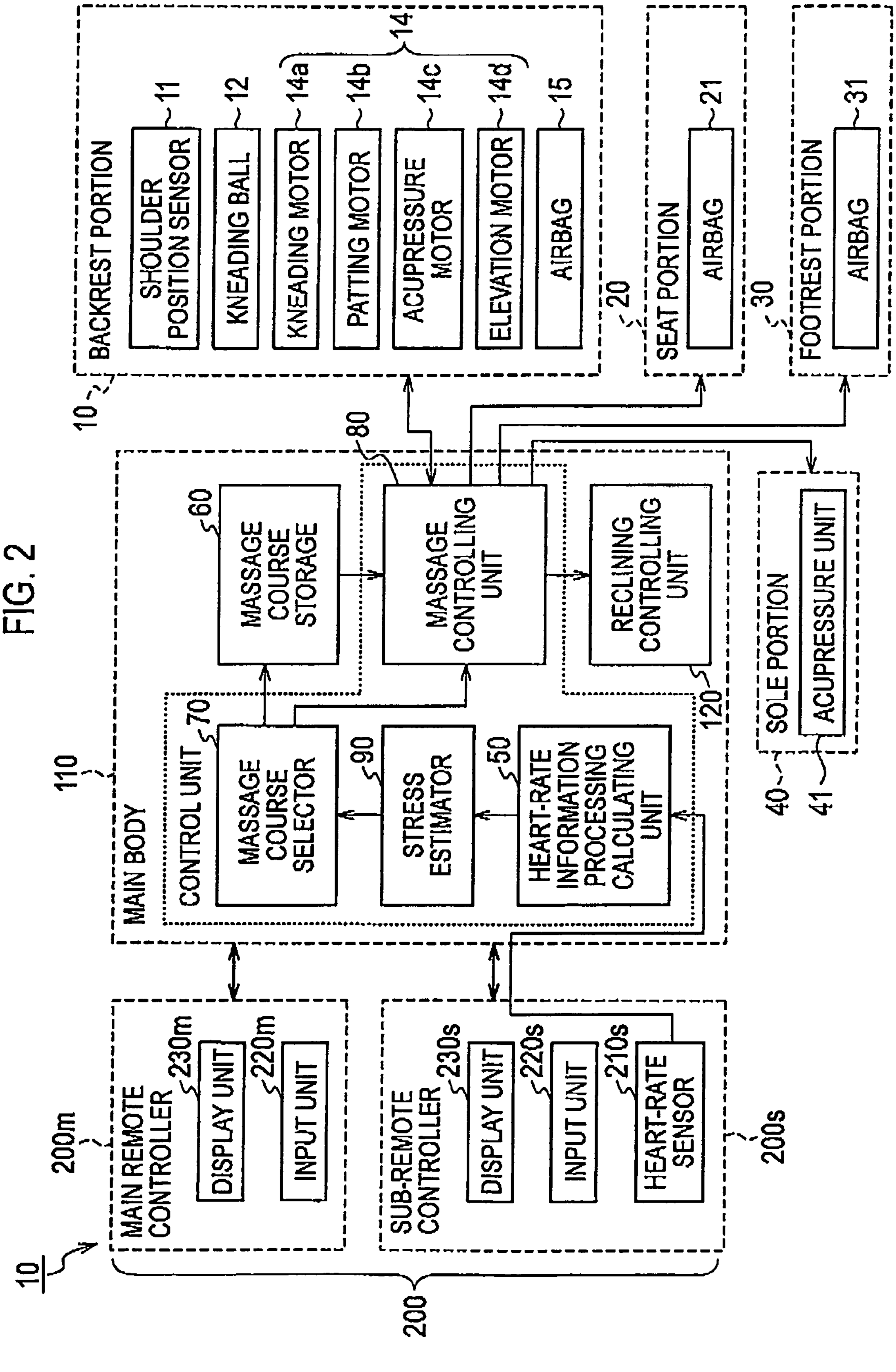
10
BACKREST PORTION
SHOULDER POSITION SENSOR
11
KNEADING BALL
12
KNEADING MOTOR
14a
PATTING MOTOR
14b
ACUPRESSURE MOTOR
14c
ELEVATION MOTOR
14d
14
AIRBAG
15
20
SEAT PORTION
AIRBAG
21
30
FOOTREST PORTION
AIRBAG
31
110
MAIN BODY
CONTROL UNIT
80
60
MASSAGE COURSE STORAGE
MASSAGE CONTROLLING UNIT
RECLINING CONTROLLING UNIT
120
70
MASSAGE COURSE SELECTOR
90
STRESS ESTIMATOR
50
HEART-RATE INFORMATION PROCESSING CALCULATING UNIT
SOLE PORTION
ACUPRESSURE UNIT
40
41
200m
MAIN REMOTE CONTROLLER
230m
DISPLAY UNIT
220m
INPUT UNIT
SUB-REMOTE CONTROLLER
230s
DISPLAY UNIT
220s
INPUT UNIT
210s
HEART-RATE SENSOR
200s
200

FIG. 3

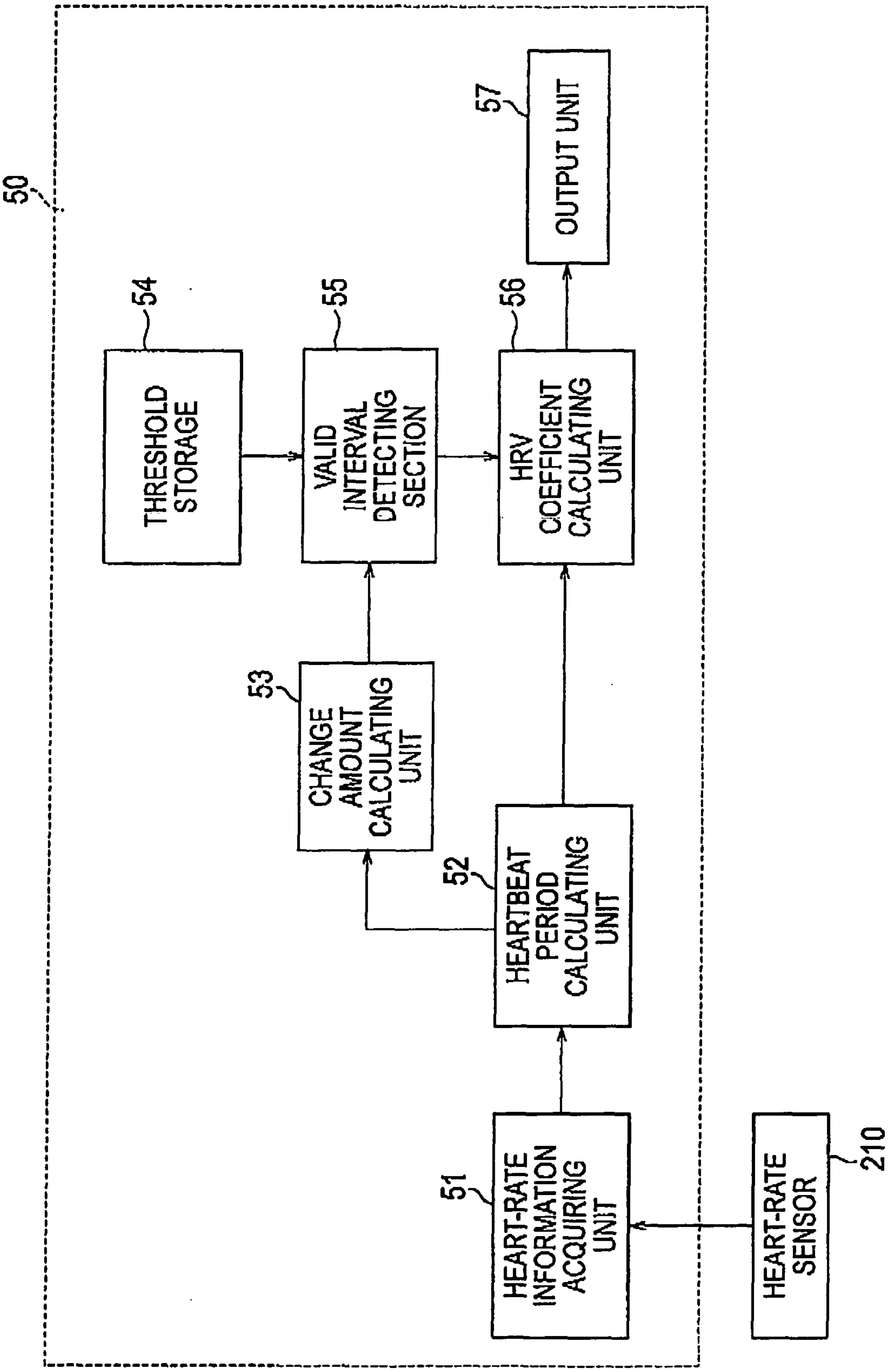
50
THRESHOLD STORAGE
54
VALID INTERVAL DETECTING SECTION
55
HRV COEFFICIENT CALCULATING UNIT
56
OUTPUT UNIT
57
CHANGE AMOUNT CALCULATING UNIT
53
HEARTBEAT PERIOD CALCULATING UNIT
52
HEART-RATE INFORMATION ACQUIRING UNIT
51
HEART-RATE SENSOR
210

FIG. 4A

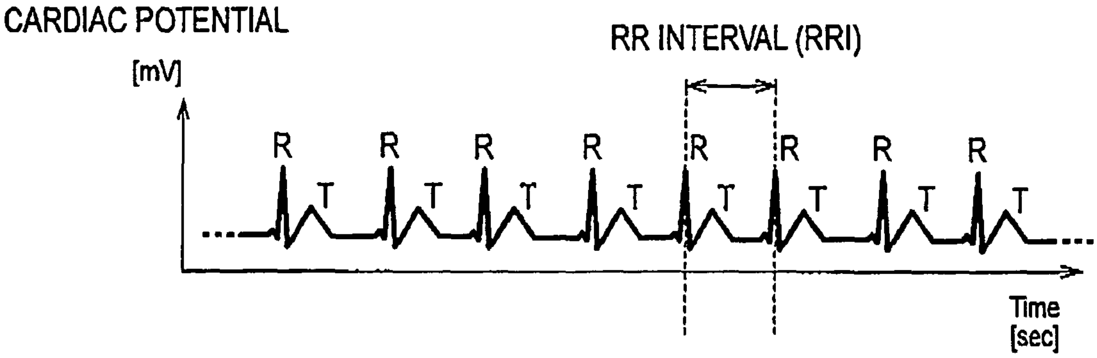


FIG. 4B

HEART-RATE VARIABILITY WAVEFORM

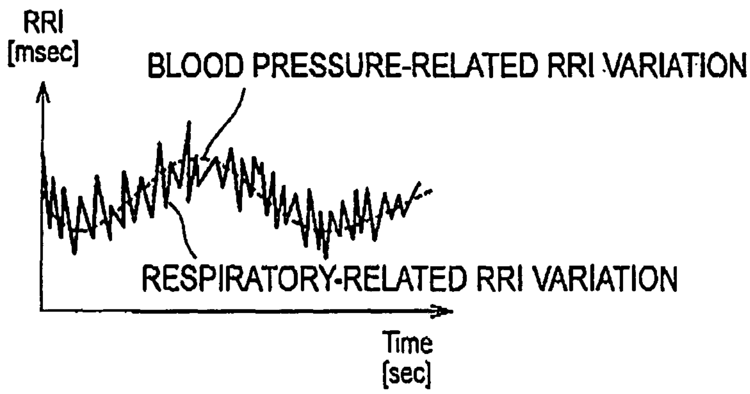

FIG. 4C

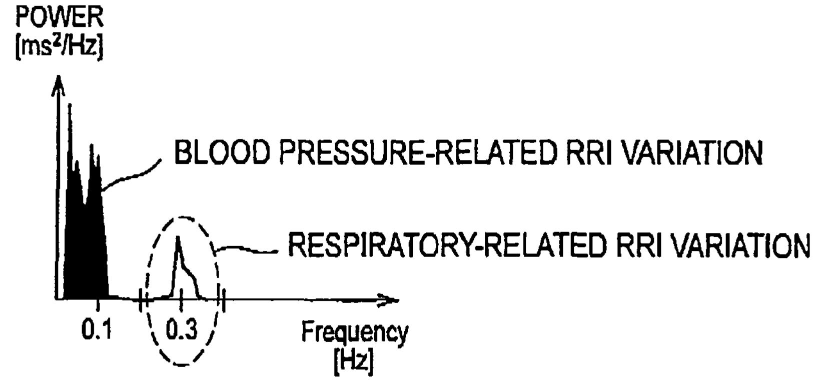


FIG. 4D

HEART-RATE VARIABILITY WAVEFORM
(ILLUSTRATED ONLY IN CONSIDERATION OF RESPIRATORY-RELATED RRI VARIATION)

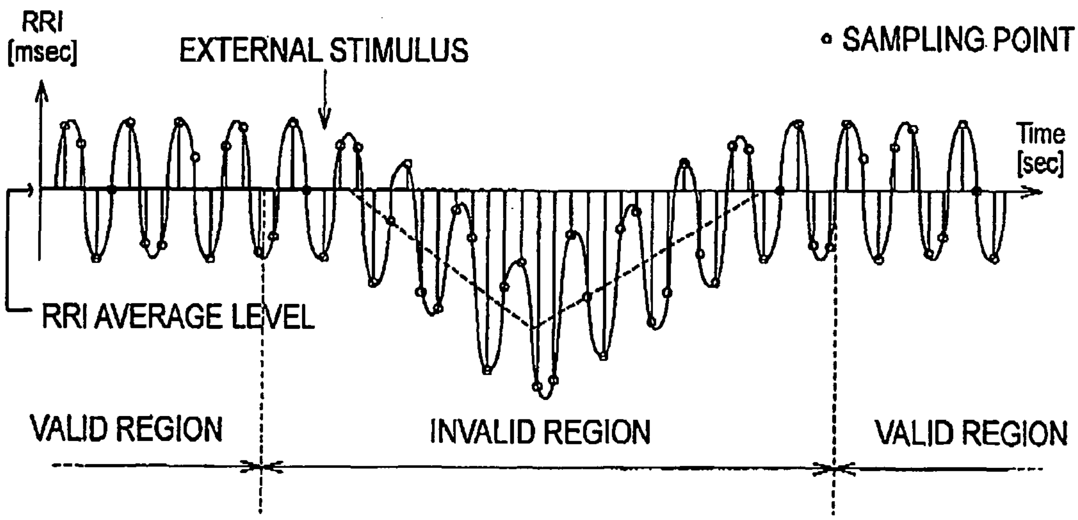

FIG. 5A

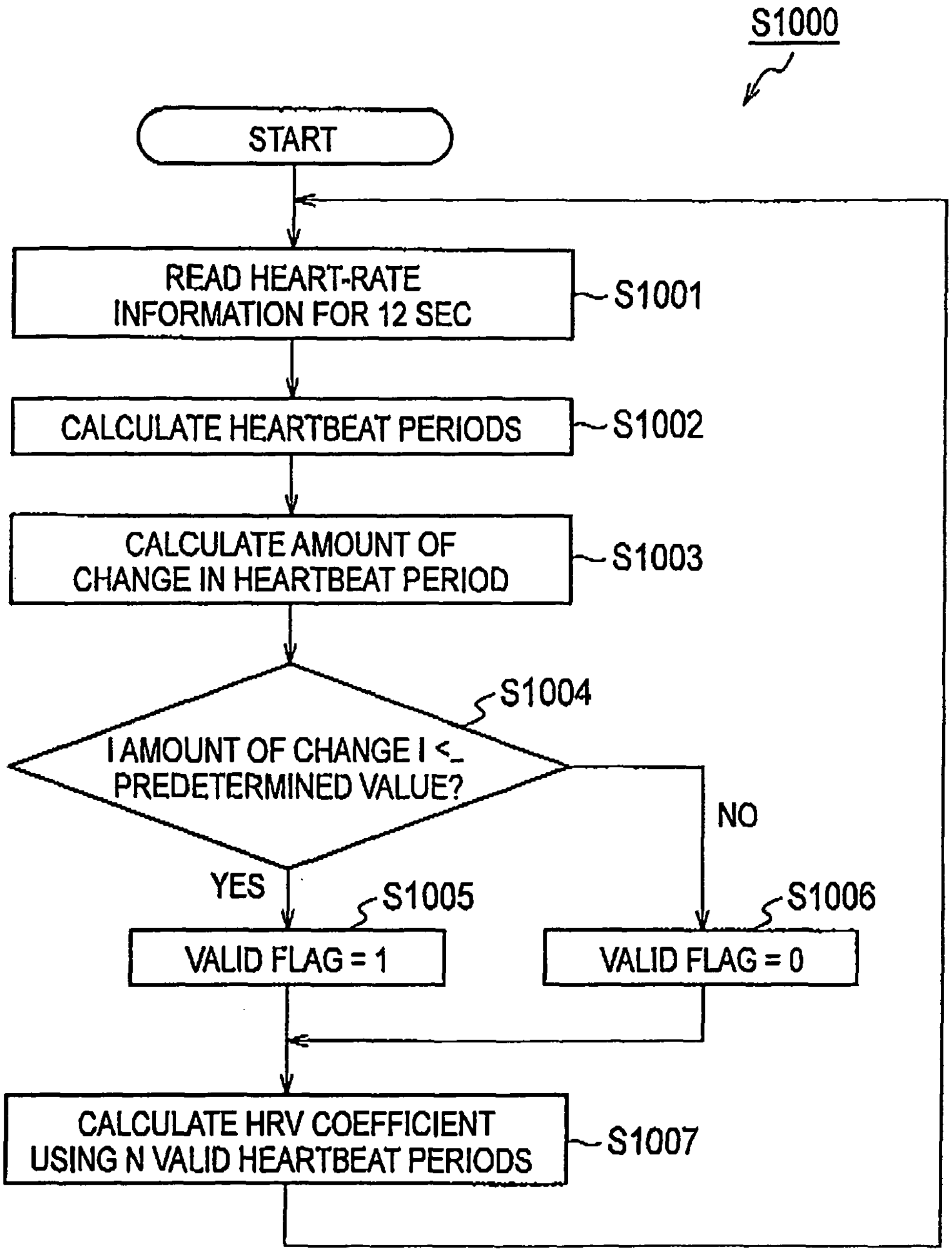
S1000
START
READ HEART-RATE INFORMATION FOR 12 SEC
S1001
CALCULATE HEARTBEAT PERIODS
S1002
CALCULATE AMOUNT OF CHANGE IN HEARTBEAT PERIOD
S1003
S1004
I AMOUNT OF CHANGE I ≤ PREDETERMINED VALUE?
NO
YES
S1005
S1006
VALID FLAG = 1
VALID FLAG = 0
CALCULATE HRV COEFFICIENT USING N VALID HEARTBEAT PERIODS
S1007

FIG. 5B

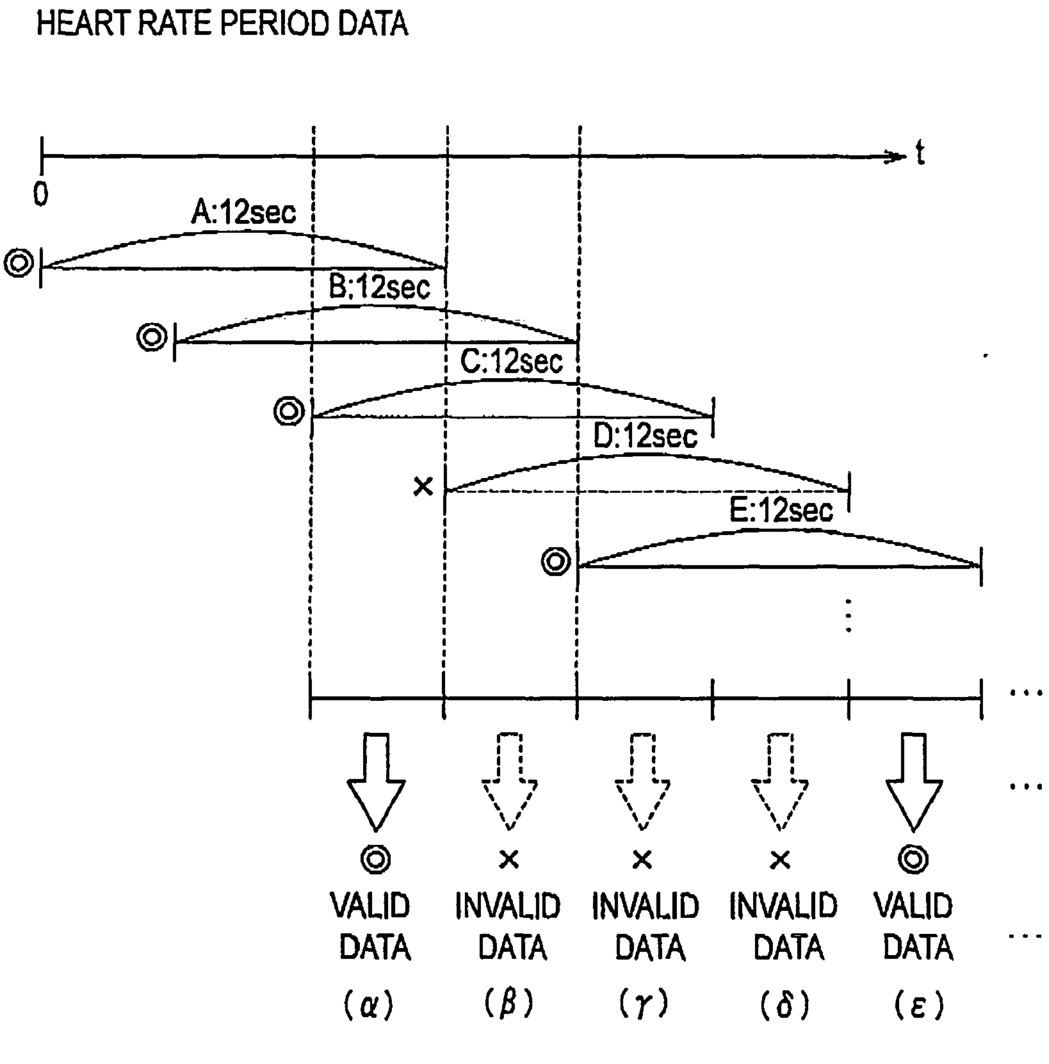
HEART RATE PERIOD DATA
t
0
A:12sec
B:12sec
C:12sec
D:12sec
E:12sec
VALID DATA (α)
INVALID DATA (β)
INVALID DATA (γ)
INVALID DATA (δ)
VALID DATA (ε)

FIG. 6

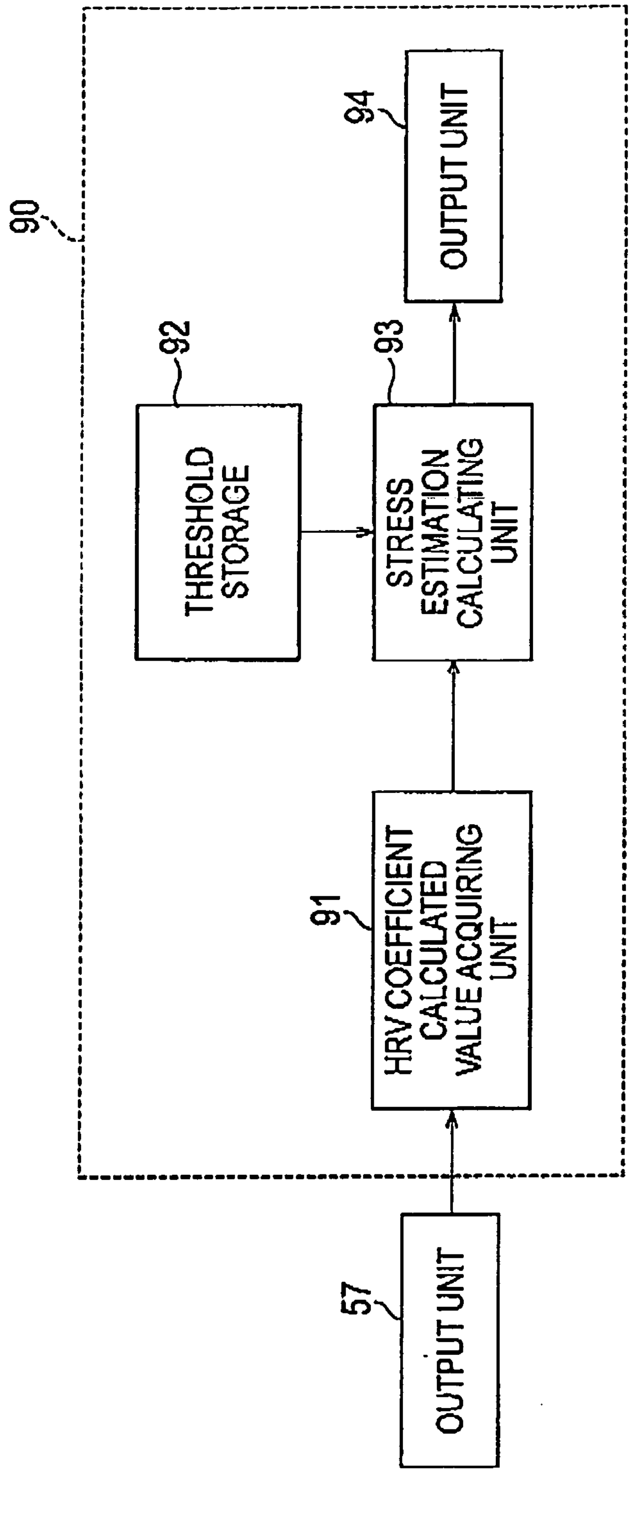
90
91
HRV COEFFICIENT CALCULATED VALUE ACQUIRING UNIT
92
THRESHOLD STORAGE
93
STRESS ESTIMATION CALCULATING UNIT
94
OUTPUT UNIT
57
OUTPUT UNIT

FIG. 7

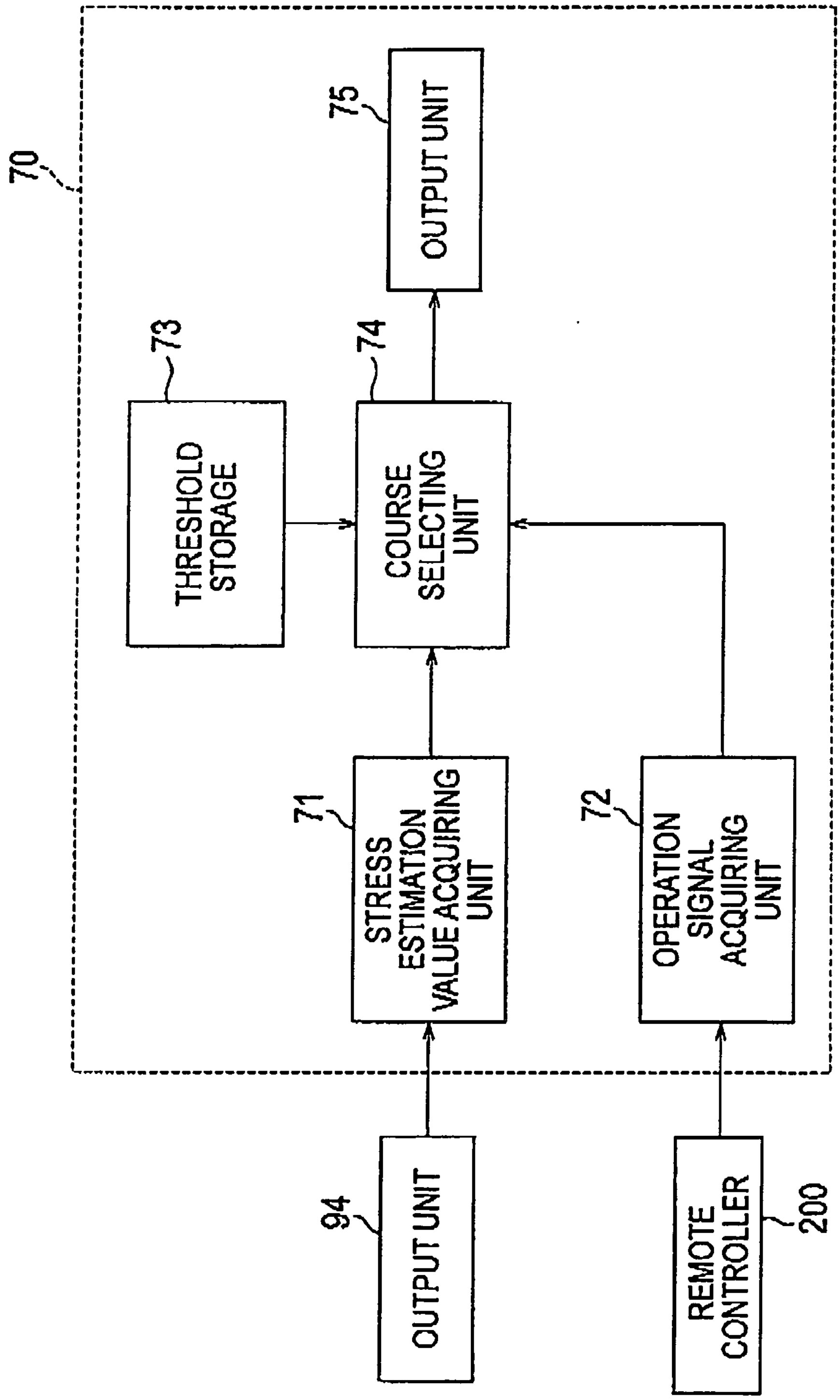
70
73
THRESHOLD STORAGE
74
COURSE SELECTING UNIT
75
OUTPUT UNIT
71
STRESS ESTIMATION VALUE ACQUIRING UNIT
72
OPERATION SIGNAL ACQUIRING UNIT
94
OUTPUT UNIT
REMOTE CONTROLLER
200

FIG. 8

| MASSAGE COURSE | STRESS ESTIMATION VALUE | STRESS ESTIMATION RESULT FOR COURSE SELECTION | CVRR RANGE | STRESS REDUCTION | HRV COEFFICIENT CALCULATED VALUE | | INCREASE IN HRV COEFFICIENT |
|---|---|---|---|---|---|---|---|
| | | | | | START OF COURSE | END OF COURSE | |
| COURSE A | 3 | VERY HIGH STRESS | BELOW 3.5% | LARGE | 4.3 | 4.8 | 0.5 |
| COURSE B | 2 | HIGH STRESS | AT OR ABOVE 3.5%, BELOW 4.5% | MEDIUM | 3.9 | 4.1 | 0.2 |
| COURSE C | 1 | LOW STRESS | AT OR ABOVE 4.5% | SMALL | 4.3 | 4.0 | - 0.3 |

FIG. 9

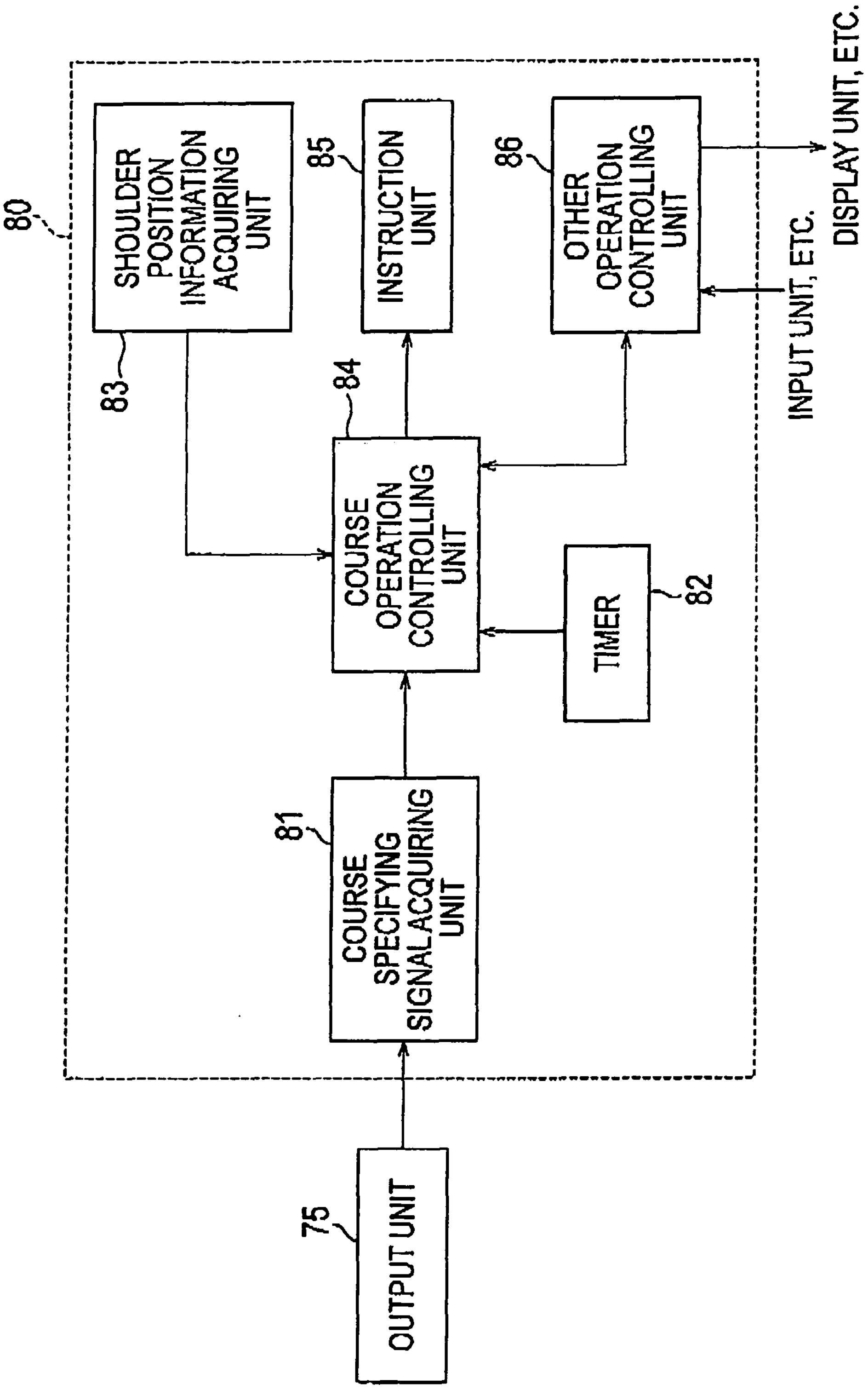
80
SHOULDER POSITION INFORMATION ACQUIRING UNIT
83
85
INSTRUCTION UNIT
86
OTHER OPERATION CONTROLLING UNIT
DISPLAY UNIT, ETC.
INPUT UNIT, ETC.
84
COURSE OPERATION CONTROLLING UNIT
TIMER
82
81
COURSE SPECIFYING SIGNAL ACQUIRING UNIT
75
OUTPUT UNIT

FIG. 10

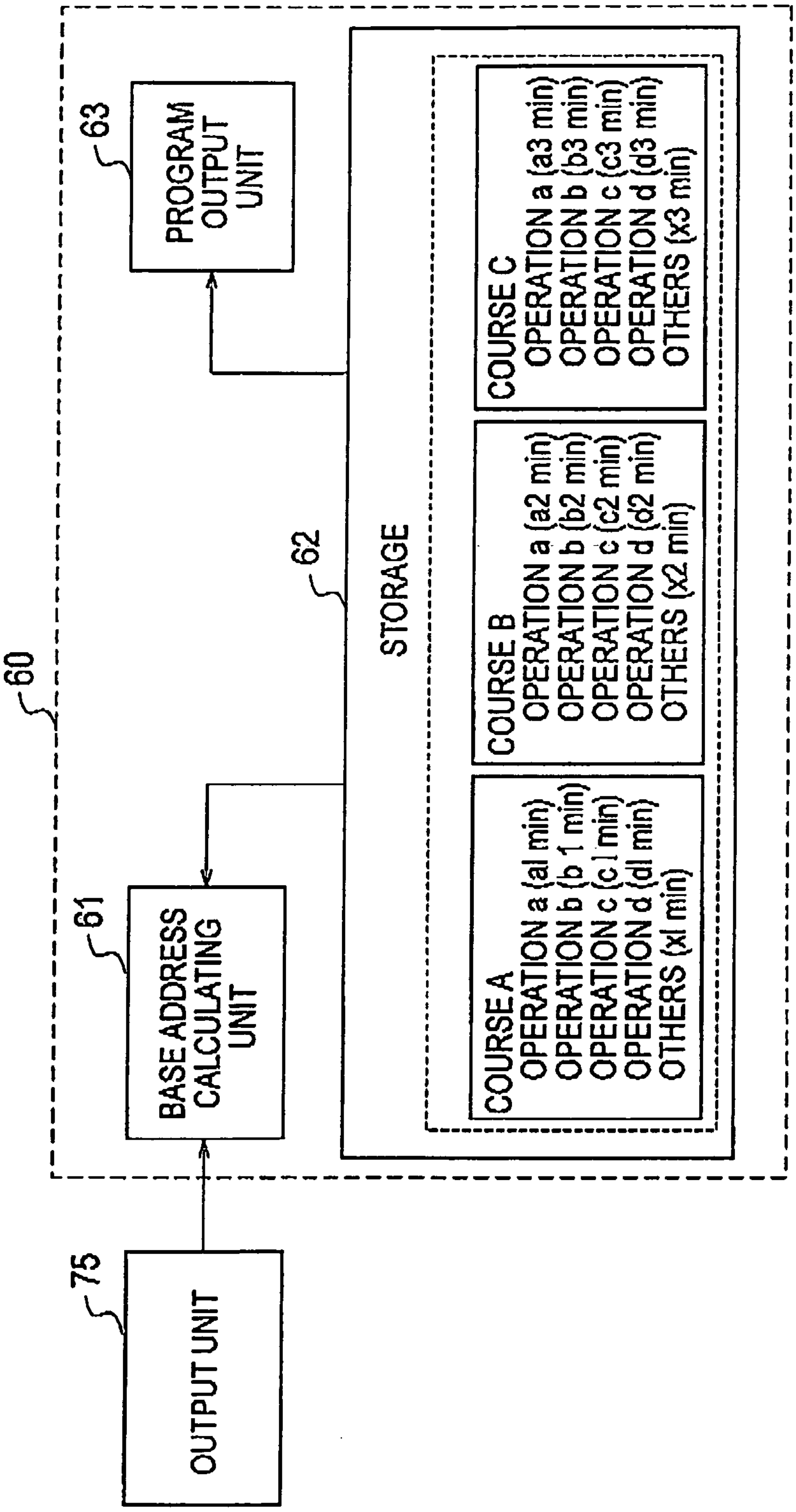
60
61
BASE ADDRESS CALCULATING UNIT
62
STORAGE
63
PROGRAM OUTPUT UNIT
75
OUTPUT UNIT
COURSE A
OPERATION a (al min)
OPERATION b (b 1 min)
OPERATION c (c l min)
OPERATION d (dl min)
OTHERS (xl min)
COURSE B
OPERATION a (a2 min)
OPERATION b (b2 min)
OPERATION c (c2 min)
OPERATION d (d2 min)
OTHERS (x2 min)
COURSE C
OPERATION a (a3 min)
OPERATION b (b3 min)
OPERATION c (c3 min)
OPERATION d (d3 min)
OTHERS (x3 min)

FIG. 11

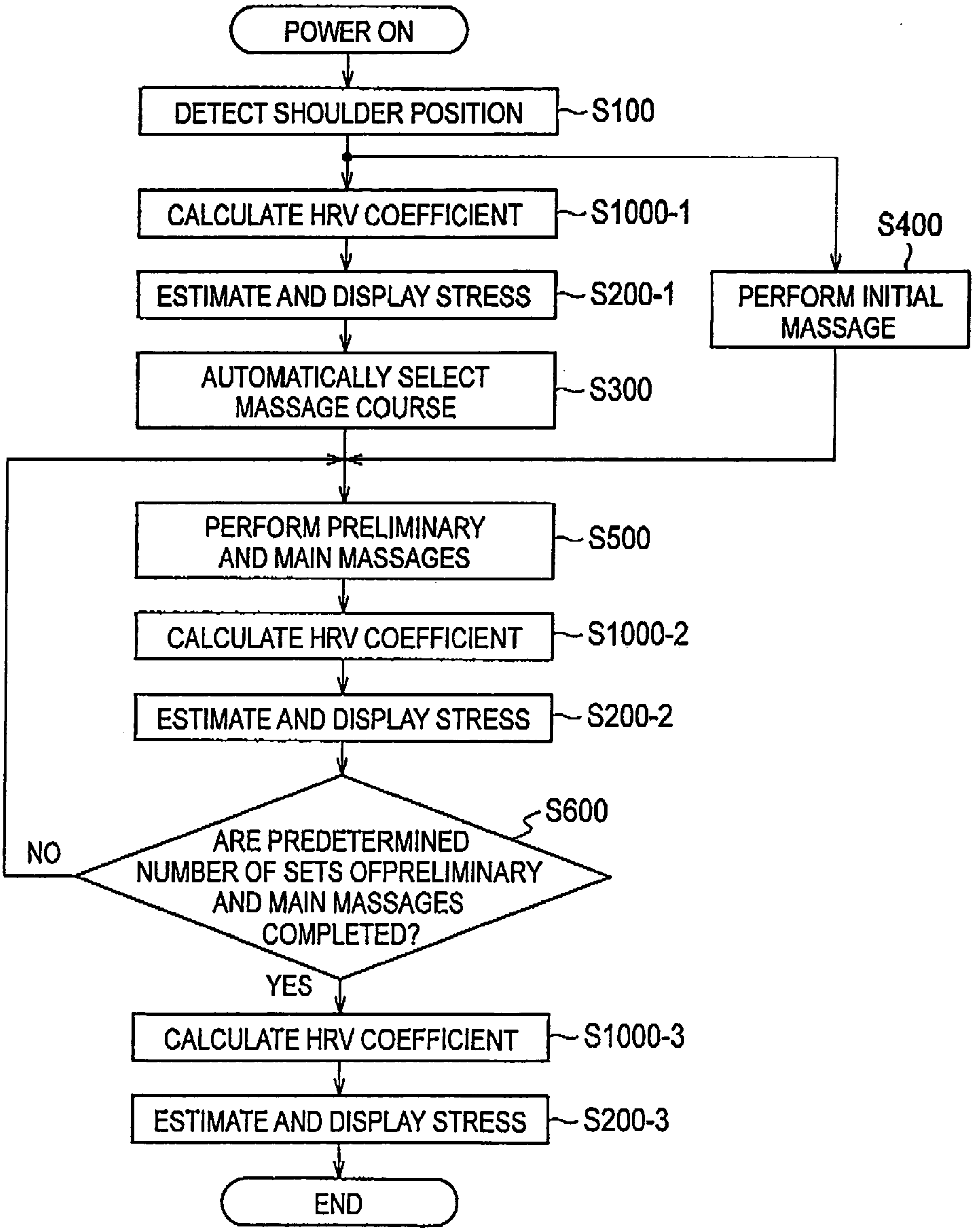
POWER ON
DETECT SHOULDER POSITION
S100
CALCULATE HRV COEFFICIENT
S1000-1
S400
ESTIMATE AND DISPLAY STRESS
S200-1
PERFORM INITIAL MASSAGE
AUTOMATICALLY SELECT MASSAGE COURSE
S300
PERFORM PRELIMINARY AND MAIN MASSAGES
S500
CALCULATE HRV COEFFICIENT
S1000-2
ESTIMATE AND DISPLAY STRESS
S200-2
S600
NO
ARE PREDETERMINED NUMBER OF SETS OFPRELIMINARY AND MAIN MASSAGES COMPLETED?
YES
CALCULATE HRV COEFFICIENT
S1000-3
ESTIMATE AND DISPLAY STRESS
S200-3
END

FIG. 12

HEART RATE

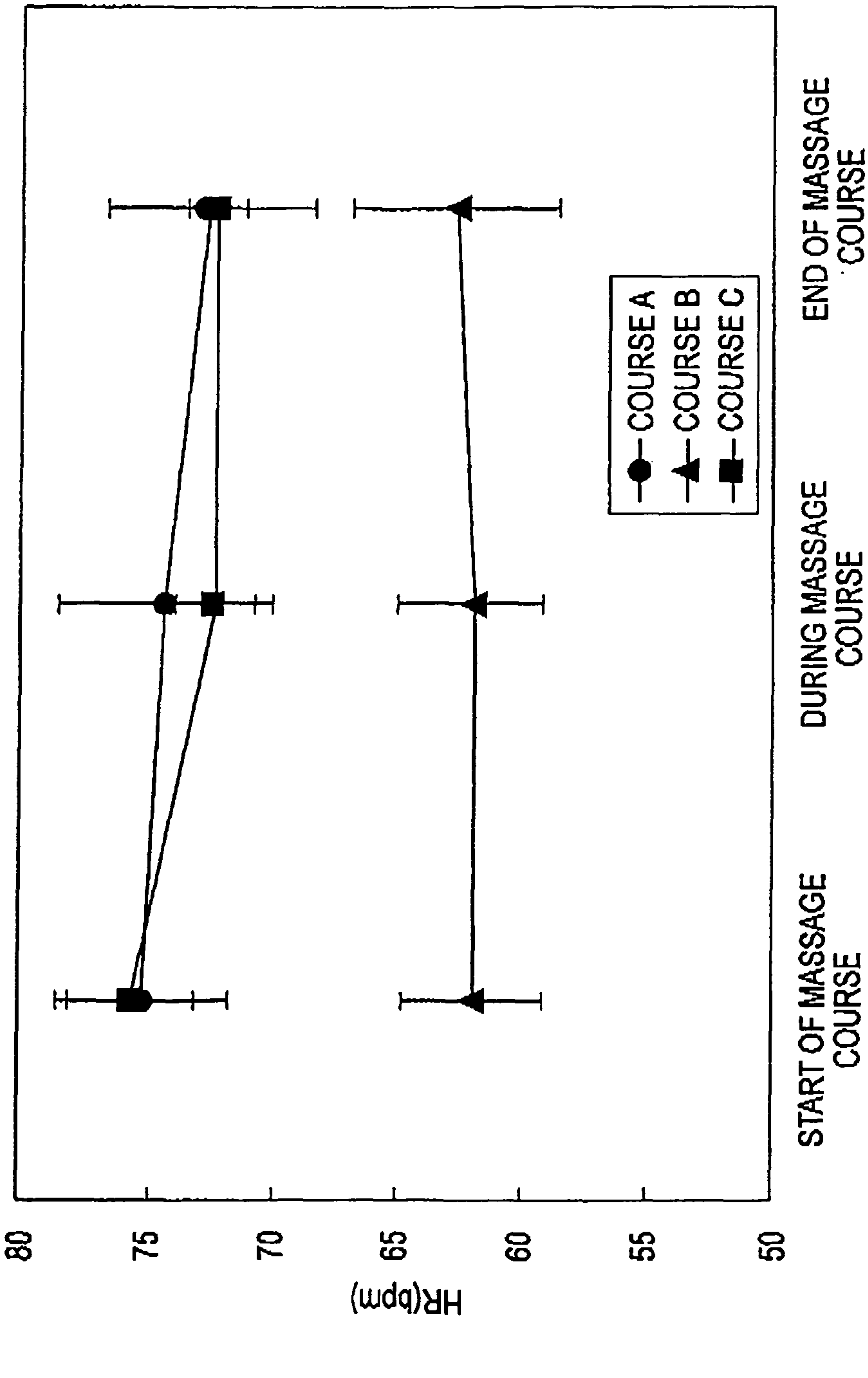

FIG. 13

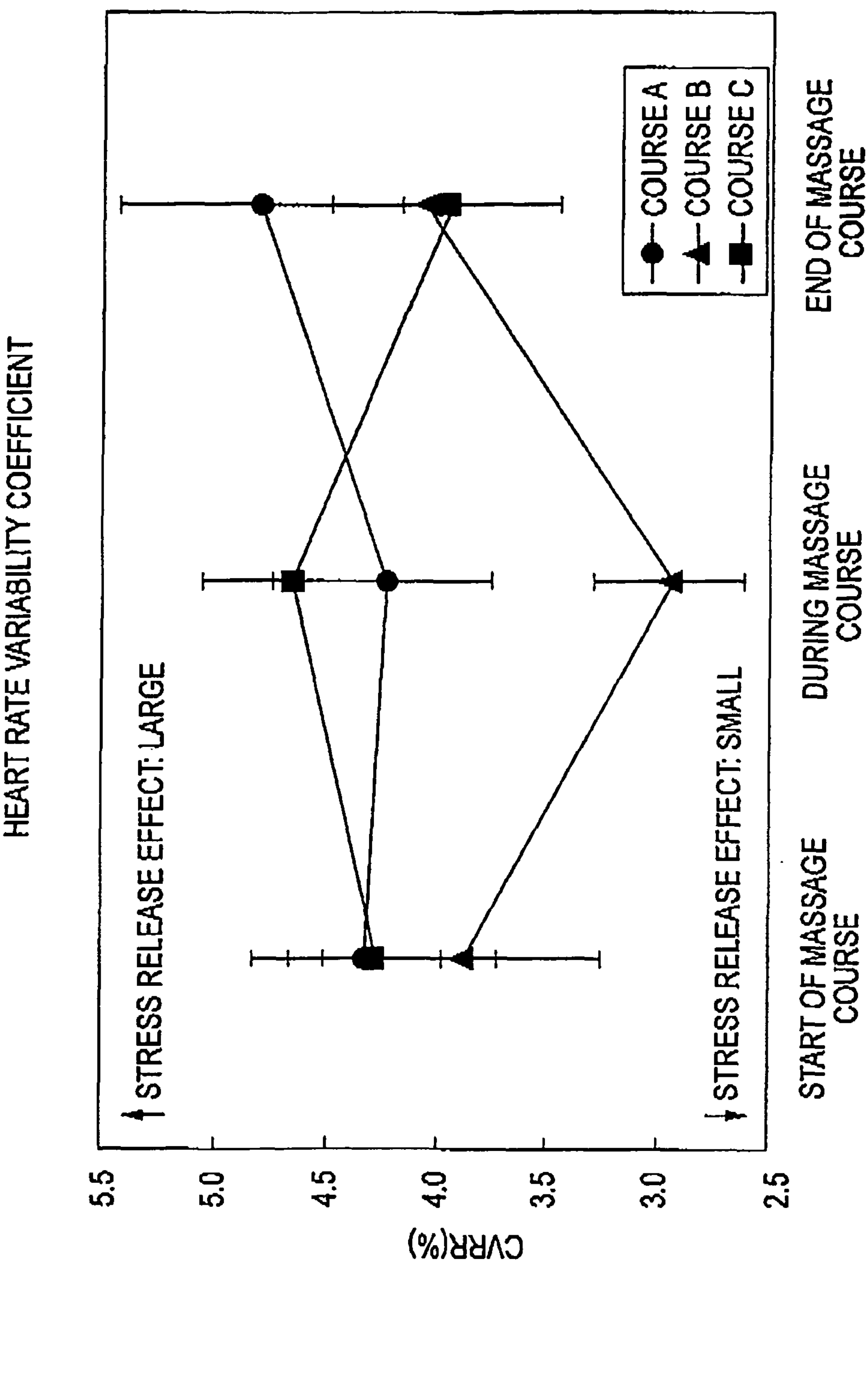
HEART RATE VARIABILITY COEFFICIENT
CVRR(%)
5.5
5.0
4.5
4.0
3.5
3.0
2.5
STRESS RELEASE EFFECT: LARGE
STRESS RELEASE EFFECT: SMALL
START OF MASSAGE COURSE
DURING MASSAGE COURSE
END OF MASSAGE COURSE
COURSE A
COURSE B
COURSE C

Europäisches Patentamt
European Patent Office
Office européen des brevets

# EUROPEAN SEARCH REPORT

Application Number
EP 10 25 0531

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/269652 A1 (REINER ROBERT HOWARD [US]) 30 October 2008 (2008-10-30) * paragraphs [0002], [0004], [0005], [0009], [0010], [0015], [0018] - [0021], [0028]; figures * | 1-5 | INV. A61H23/02 A61H23/04 A61H1/00 A61B5/0452 A61M21/02 |
| X | US 5 993 401 A (INBE HIROYUKI [JP] ET AL) 30 November 1999 (1999-11-30) * column 1, line 49 - column 2, line 7 * * column 6, lines 7-59; figures * | 1-5 | |
| X<br>Y | US 2002/068887 A1 (KIKUMOTO MAKOTO [JP] ET AL) 6 June 2002 (2002-06-06) * paragraphs [0013] - [0017], [0021]; figures * | 1,4,5<br>2,3 | |
| Y | US 2006/030907 A1 (MCNEW BARRY [US]) 9 February 2006 (2006-02-09) * paragraphs [0017], [0018], [0066], [0067]; claims 7,20 * | 2,3 | |
| | | | TECHNICAL FIELDS SEARCHED (IPC) |
| | | | A61H A61B A61M |

The present search report has been drawn up for all claims

| Place of search | Date of completion of the search | Examiner |
|---|---|---|
| Munich | 7 July 2010 | Fischer, Elmar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 10 25 0531

This annex lists the patent family members relating to the patent documents cited in the above-mentioned European search report.
The members are as contained in the European Patent Office EDP file on
The European Patent Office is in no way liable for these particulars which are merely given for the purpose of information.

07-07-2010

| Patent document cited in search report | Publication date | Patent family member(s) | Publication date |
|---|---|---|---|
| US 2008269652 A1 | 30-10-2008 | NONE | |
| US 5993401 A | 30-11-1999 | DE 19681246 B4 | 29-04-2004 |
| | | DE 19681246 T0 | 22-01-1998 |
| | | WO 9723254 A1 | 03-07-1997 |
| US 2002068887 A1 | 06-06-2002 | KR 20020042485 A | 05-06-2002 |
| | | TW 505513 B | 11-10-2002 |
| US 2006030907 A1 | 09-02-2006 | CA 2550007 A1 | 30-06-2005 |
| | | EP 1699405 A2 | 13-09-2006 |
| | | KR 20070085052 A | 27-08-2007 |
| | | US 2007203432 A1 | 30-08-2007 |
| | | WO 2005058144 A2 | 30-06-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005341989 A